Europäisches Patentamt

European Patent Office

Office européen des brevets

(11) Numéro de publication: **0 295 156 B1**

# FASCICULE DE BREVET EUROPEEN

(45) Date de publication de fascicule du brevet: **26.04.95**

(51) Int. Cl.⁶: **C12N 15/32**, C12N 1/21, C12P 21/02, A01N 63/00, A01H 5/00

(21) Numéro de dépôt: **88401121.4**

(22) Date de dépôt: **06.05.88**

(54) **Séquences de nucléotides codant pour des polypeptides dotés d'une activité larvicide vis-à-vis de lépidoptères.**

(30) Priorité: **10.06.87 FR 8708090**

(43) Date de publication de la demande:
**14.12.88 Bulletin 88/50**

(45) Mention de la délivrance du brevet:
**26.04.95 Bulletin 95/17**

(84) Etats contractants désignés:
**AT BE CH DE ES FR GB GR IT LI LU NL SE**

(56) Documents cités:
**EP-A- 0 178 151**
**EP-A- 0 192 319**
**EP-A- 0 224 331**
**EP-A- 0 228 838**

(73) Titulaire: **INSTITUT PASTEUR**
**28, rue du Docteur Roux**
**F-75724 Paris Cédex 15 (FR)**

Titulaire: **CENTRE NATIONAL DE LA RECHER-CHE SCIENTIFIOUE**
**15, quai Anatole France**
**F-75007 Paris (FR)**

Titulaire: **INSTITUT NATIONAL DE LA RECHER-CHE AGRONOMIOUE**
**145, rue de l'Université**
**F-75341 Paris Cédex 07 (FR)**

(72) Inventeur: **Sanchis, Vincent**
**15 avenue Toulouse Lautrec**
**F-78390 Bois d'Arcy (FR)**
Inventeur: **Lereclus, Didier**
**16 bis rue Lauriston**
**F-75116 Paris (FR)**
Inventeur: **Menou, Ghislaine**
**22 rue Rosenwald**
**F-75015 Paris (FR)**

**MOLECULAR BIOLOGY OF MICROBIOL DIF-FERENTATION, PROCEEDINGS OF THE NIN-THINTERNATIONAL SPORE CONFERENCE,** Asilomar, California, 3-6 septembre 1984, pages217-224, 1985, Washington, DC, US; A. KLIER et al.: "Cloning and expression inEscherichia coli of the crystal protein gene from bacillus thuringiensis strainaizawa 7-29 and comparison of the structural organization of genes fromdifferent serotypes"

Inventeur: **Lecadet, Marguerite-Marie**
**10 rue Nicolas Charlet**
**F-75015 Paris (FR)**
Inventeur: **Martouret, Daniel**
**6 Square de l'Hôtel de Ville**
**F-78210 Saint-Cyr-l'Ecole (FR)**
Inventeur: **Dedonder, Raymond**
**3 allée des Pépinières**
**F-92290 Chatenay-Malabry (FR)**

74) Mandataire: **Peaucelle, Chantal et al**
**S.C. Ernest Gutmann - Yves Plasseraud**
**67, boulevard Haussmann**
**F-75008 Paris (FR)**

## Description

L'invention a pour objet des séquences de nucléotides codant pour des polypeptides dotés d'une activité larvicide vis-à-vis de lépidoptères.

Elle vise plus spécialement des moyens, en particulier des séquences nucléotidiques, des polypeptides ou encore des vecteurs, ou des souches bactériennes, modifiés par ces séquences et exprimant des polypeptides permettant de préparer des compositions larvicides actives vis-à-vis de lépidoptères, de préférence vis-à-vis de Spodoptera littoralis (ci-après S.littoralis) ou Mamestra brassicae (ci-après désignée par M.brassicae) ou capables de transformer les plantes à traiter en leur conférant ce type d'activité.

On sait que la plupart des isolats de B.thuringiensis présentent une activité toxique à l'égard de larves de plus de cent espèces de lépidoptères.

Cette activité résulte, de la capacité des souches de B.thuringiensis à synthètiser, au moment de la sporulation, des inclusions cristallines de nature protéique, ou δ-endotoxines, sous le contrôle d'un ou plusieurs types de gènes.

On a montré que l'activité de ces polypeptides est contenue dans la moitié NH$_2$-terminale ou N-terminale de la protéine.

Les travaux réalisés ont montré la spécificité élevée des δ-endotoxines vis-à-vis de larves d'une espèce donnée.

En raison de cette spécificité élevée, de nombreuses espèces de lépidoptères, notamment de la famille des Noctuelles, ne réagissent que faiblement aux préparations commerciales de B.thuringiensis disponibles.

Il en est ainsi en particulier de l'espèce S.littoralis, insecte polyphage qui constitue le principal parasite du coton et d'autres cultures industriellement importantes. Parmi ces cultures, on citera le maïs, le ricin, le tabac, l'arachide, des plantes fourragères, telles que le trèfle ou la luzerne, ou encore des produits maraîchers comme le chou ou la tomate.

On conçoit donc l'intérêt de disposer de moyens ciblant spécifiquement et efficacement la famille des Noctuelles et notamment S.littoralis ou M.brassicae.

Les gènes de δ-endotoxines identifiés à ce jour, ne codent pas pour un polypeptide actif préférentiellement à l'égard de S.littoralis.

Dans une publication dans "Molecular Biology of Microbial Differentiation" p.217-222, Sept. 1984, Klier et al. ont décrit l'expression dans E.coli d'un fragment BamHI de 13 kb d'un gène d'une protéine du crystal de B.thuringiensis aizawai 7.29. Ce fragment d'ADN exprime un polypeptide d'environ 130 kDa.

La recherche par les inventeurs d'une séquence de nucléotides codant pour un polypeptide actif de préférence vis-à-vis des Noctuelles, plus spécialement vis-à-vis de S.littoralis, les a conduits à étudier les isolats naturels de deux souches de B.thuringiensis dont l'activité larvicide sur S.littoralis apparaît plus élevée que celle des préparations industrielles faites à partir d'autres souches de B.thuringiensis.

Il s'agit des souches aizawai 7-29 et entomocidus 6-01.

L'étude de ces isolats a permis de mettre en évidence l'existence de plusieurs gènes de δ-endotoxines de structures différentes et de spécificités différentes, dont deux gènes préférentiellement actifs contre P.brassicae mais peu actifs vis-à-vis de la Noctuelle du coton et un gène inactif vis-à-vis de P.brassicae et de S.littoralis.

En étudiant l'ADN total de ces isolats et en effectuant des hybridations appropriées, suivies du clonage des fragments identifiés par hybridation, les inventeurs ont constaté qu'il est possible d'isoler des séquences nucléotidiques impliquées dans des gènes de δ-endotoxines codant pour des polypeptides actifs, de préférence contre S.littoralis.

L'invention a donc pour but de fournir des séquences nucléotidiques capables de coder pour au moins la partie NH$_2$-terminale d'une δ-endotoxine toxique contre les Noctuelles, de préférence contre S.littoralis ou M.brassicae.

Elle a également pour but de fournir un polypeptide toxique à l'égard des Noctuelles.

L'invention vise en outre un procédé d'obtention d'une telle séquence et d'un polypeptide présentant l'activité recherchée ainsi que les moyens intermédiaires tels que vecteurs et souches bactériennes utilisables pour l'obtention du polypeptide.

L'invention vise de plus les applications de ces séquences et polypeptides pour l'élaboration de compositions larvicides l'égard des Noctuelles, en particulier de S.littoralis et pour la transformation des plantes susceptibles d'être infectées par ces larves.

L'invention concerne une séquence de nucléotides codant pour au moins une partie de la région N-terminale d'un polypeptide toxique de façon spécifique vis-à-vis des larves de lépidoptères de la famille des Noctuelles, de préférence vis-à-vis de S.littoralis, caractérisée par sa capacité d'hybridation avec un gène capable d'exprimer un polypeptide toxique vis-à-vis de larves de S.littoralis.

3

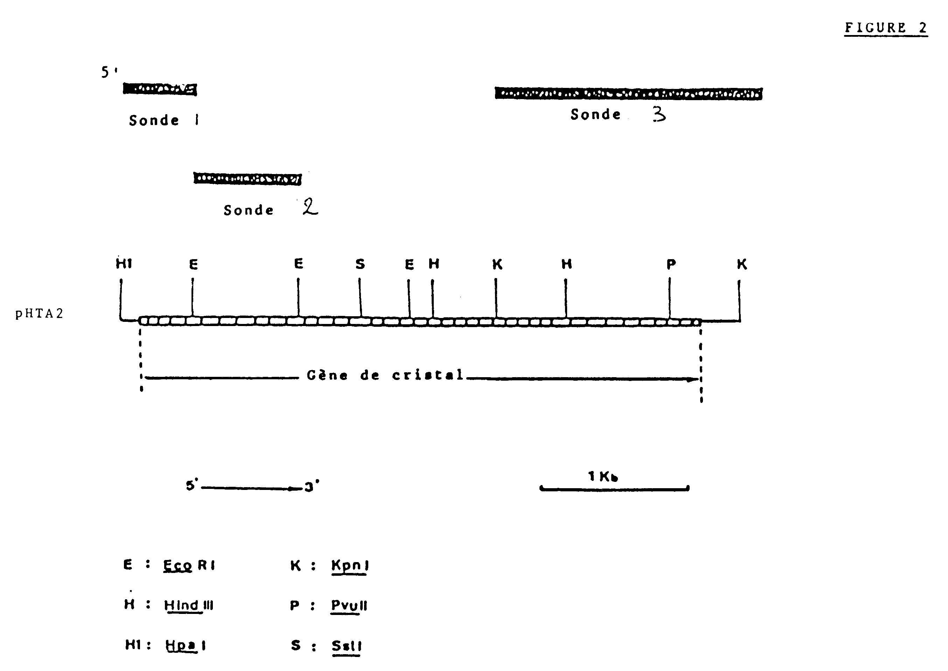

Selon un autre aspect de l'invention, la séquence nucléotidique est caractérisée en ce qu'elle est portée par une séquence de nucléotides d'environ 3kb telle qu'obtenue par recombinaison génétique in vitro de séquences de nucléotides de B.thuringiensis capables de s'hybrider avec des sondes 1, 2 et 3 de pHTA2 rapportées sur la figure 2. Le fragment de 3kb correspond plus spécialement au fragment de restriction HindIII-PstI.

Les séquences de nucléotides de l'invention sont, en outre, caractérisées en ce qu'elles comportent des sites dans l'ordre suivant : HindIII - HincII - BglII KpnI - HindIII - PstI .

De manière préférée, ces séquences de nucléotides sont obtenues par recombinaison génétique in vitro de séquences d'ADN provenant d'au moins une souche de B.thuringiensis. Dans une variante de réalisation de l'invention, deux souches diiférentes de B.thuringiensis sont mises en oeuvre.

Des souches de B.thuringiensis particulièrement appropriées pour l'obtention de ces séquences de nucléotides sont les souches correspondant à aizawai 7-29 et entomocidus 6-01, déposées le 21 Avril 1987 respectivement sous les n° 1-661 et n° I-660 à la Collection nationale de Culture de Microorganismes (C.N.C.M.) à Paris.

D'une manière avantageuse, les séquences de nucléotides de l'invention codent pour un polypeptide capable de former un complexe immunologique avec des anticorps dirigés contre des polypeptides présentant l'activité larvicide à l'égard de S.littoralis.

Une séquence de nucléotides selon l'invention est caractérisée en ce qu'elle a la capacité de s'hybrider avec une sonde formée à partir de la séquence (I) présentant l'enchaînement suivant :

```
      52
GTC TAC TTG ACA GGG GTA GGA ACA TAA TCG GTC AAT TTT AAA TAT GGG GCA TAT ATT GAT
      112
ATT TTA TAA AAT TTG TTA CGT TTT TTG TAT TTT TTC ATA AGA TGT GTC ATA TGT ATT AAA
      172
TCG TGG TAA TGA AAA ACA GTA TCA AAC TAT CAG AAC TTT GGT AGT TTA ATA AAA AAA CGG
      232
AGG TAT TTT ATG GAG GAA AAT AAT CAA AAT CAA TGC ATA CCT TAC AAT TGT TTA AGT AAT
      292
CCT GAA GAA GTA CTT TTG GAT GGA GAA CGG ATA TCA ACT GGT AAT TCA TCA ATT GAT ATT
      352
TCT CTG TCA CTT GTT CAG TTT CTG GTA TCT AAC TTT GTA CCA GGG GGA GGA TTT TTA GTT
      412
GGA TTA ATA GAT TTT GTA TGG GGA ATA GTT GGC CCT TCT CAA TGG GAT GCA TTT CTA GTA
      472
CAA ATT GAA CAA TTA ATT AAT GAA AGA ATA GCT GAA TTT GCT AGG AAT GCT GCT ATT GCT
      532
AAT TTA GAA GGA TTA GGA AAC AAT TTC AAT ATA TAT GTG GAA GCA TTT AAA GAA TGG GAA
      592
GAA GAT CCT AAT AAT CCA GAA ACC AGG ACC AGA GTA ATT GAT CGC TTT CGT ATA CTT GAT
      652
GGG CTA CTT GAA AGG GAC ATT CCT TCG TTT CGA ATT TCT GGA TTT GAA GTA CCC CTT TTA
      712
TCC GTT TAT GCT CAA GCG GCC AAT CTG CAT CTA GCT ATA TTA AGA GAT TCT GTA ATT TTT
      772
GGA GAA AGA TGG GGA TTG ACA ACG ATA AAT GTC AAT GAA AAC TAT AAT AGA CTA ATT AGG
      832
CAT ATT GAT GAA TAT GCT GAT CAC TGT GCA AAT ACG TAT AAT CGG GGA TTA AAT AAT TTA
      892
CCG AAA TCT ACG TAT CAA GAT TGG ATA ACA TAT AAT CGA TTA CGG AGA GAC TTA ACA TTG
      952
ACT GTA TTA GAT ATC GCC GCT TTC TTT CCA AAC TAT GAC
```

Des séquences de nucléotides codant pour au moins une partie de la région N-terminale d'un polypeptide toxique de manière spécifique vis-à-vis de larves de lépidoptères de la famille des Noctuelles, de préférence vis-à-vis de S.littoralis sont caractérisées en ce qu'elles comprennent l'enchaînement (I) défini ci-dessus.

D'une manière avantageuse, la séquence de nucléotides caractérisée par l'enchaînement défini ci-dessus code pour une partie d'un polypeptide ayant une activité larvicide vis-à-vis de S.littoralis plus élevée que celle des polypeptides codés par des isolats naturels actuellement connus pour leurs effets vis-à-vis de S.littoralis.

L'étude de cette séquence de nucléotides montre qu'elle est caractérisée par un codon d'initiation ATG situé en position 241 à partir duquel une phase ouverte de lecture de 750 nucléotides a été identifiée.

Cette séquence est également caractérisée par un site d'attachement des ribosomes GGAGG en positions 230 à 234.

Selon un autre aspect, la séquence de nucléotides de l'invention est caractérisée en ce qu'elle comporte, en amont du codon ATG, une séquence allant du nucléotide en position 137 au nucléotide en position 177, fortement homologue à la région trouvée par Wong et al. (1983) et décrit dans (16) en amont du gène du cristal de la souche kurstaki HD1 Dipel (BTK) et dont les auteurs ont montré qu'elle contenait trois promoteurs BtI, BtII et Ec qui sont respectivement fonctionnels chez B.thuringiensis et E.coli. L'homologie de ces séquences est d'environ 70%.

L'invention concerne également une séquence de nucléotides codant pour la séquence (II) d'acides aminés suivante :

```
MET GLU GLU ASN ASN GLN ASN GLN CYS ILE PRO TYR ASN CYS LEU SER ASN

PRO GLU GLU VAL LEU LEU ASP GLY GLU ARG ILE SER THR GLY ASN SER SER ILE ASP ILE

SER LEU SER LEU VAL GLN PHE LEU VAL SER ASN PHE VAL PRO GLY GLY GLY PHE LEU VAL

GLY LEU ILE ASP PHE VAL TRP GLY ILE VAL GLY PRO SER GLN TRP ASP ALA PHE LEU VAL

GLN ILE GLU GLN LEU ILE ASN GLU ARG ILE ALA GLU PHE ALA ARG ASN ALA ALA ILE ALA

ASN LEU GLU GLY LEU GLY ASN ASN PHE ASN ILE TYR VAL GLU ALA PHE LYS GLU TRP GLU

GLU ASP PRO ASN ASN PRO GLU THR ARG THR ARG VAL ILE ASP ARG PHE ARG ILE LEU ASP

GLY LEU LEU GLU ARG ASP ILE PRO SER PHE ARG ILE SER GLY PHE GLU VAL PRO LEU LEU

SER VAL TYR ALA GLN ALA ALA ASN LEU HIS LEU ALA ILE LEU ARG ASP SER VAL ILE PHE

GLY GLU ARG TRP GLY LEU THR THR ILE ASN VAL ASN GLU ASN TYR ASN ARG LEU ILE ARG

HIS ILE ASP GLU TYR ALA ASP HIS CYS ALA ASN THR TYR ASN ARG GLY LEU ASN ASN LEU

PRO LYS SER THR TYR GLN ASP TRP ILE THR TYR ASN ARG LEU ARG ARG ASP LEU THR LEU

THR VAL LEU ASP ILE ALA ALA PHE PHE PRO ASN TYR ASP
```

Une meilleure identification de la séquence de nucléotides isolée des souches ci-dessus, déposées à la CNCM a permis de constater que le nucléotide situé en position 273 est la guanine (G), l'acide aminé résultant du codon GTA étant alors la valine.

Or, la lecture du nucléotide correspondant à cette position 273 dans la demande FR.8708090 du 10 juin 1987 avait conduit à mentionner la thymine (T) et, comme acide aminé résultant du codon TTA, la leucine.

Une autre séquence de nucléotides de l'invention est caractérisée par sa capacité d'hybridation avec une sonde formée à partir de la séquence (III) présentant l'enchaînement suivant :

5

EP 0 295 156 B1

```
   1
GAG CTT CAA TAG AAT CTC AAA TCT CGA TGA CTG CTT AGT CTT TTT AAT ACT GTC TAC TTG ACA GGG GTA GGA ACA TAA TCG GTC AAT TTT
  91
AAA TAT GGG GCA TAT ATT GAT ATT TTA TAA AAT TTG TTA CGT TTT TTG TAT TTT TTC ATA AGA TGT GTC ATA TGT ATT AAA TCG TGG TAA
 181
TGA AAA ACA GTA TCA AAC TAT CAG AAC TTT GGT AGT TTA ATA AAA AAA CGG AGG TAT TTT ATG GAG GAA AAT AAT CAA AAT CAA TGC ATA
 271
CCT TAC AAT TGT TTA AGT AAT CCT GAA GAA GTA CTT TTG GAT GGA GAA CGG ATA TCA ACT GGT AAT TCA TCA ATT GAT ATT TCT CTG TCA
 361
CTT GTT CAG TTT CTG GTA TCT AAC TTT GTA CCA GGG GGA GGA TTT TTA GTT GGA TTA ATA GAT TTT GTA TGG GGA ATA GTT GGC CCT TCT
 451
CAA TGG GAT GCA TTT CTA GTA CAA ATT GAA CAA TTA ATT AAT GAA AGA ATA GCT GAA ATT GCT AGG AAT GCT GCT ATT GCT AAT TTA GAA
 541
GGA TTA GGA AAC AAT TTC AAT ATA TAT GTG GAA GCA TTT AAA GAA TGG GAA GAA GAT CCT AAT AAT CCA GCA ACC AGG ACC AGA GTA ATT
 631
GAT CGC TTT CGT ATA CTT GAT GGG CTA CTT GAA AGG GAC ATT CCT TCG TTT CGA ATT TCT GGA TTT GAA GTA CCC CTT TTA TCC GTT TAT
 721
GCT CAA GCG GCC AAT CTG CAT CTA GCT ATA TTA AGA GAT TCT GTA ATT TTT GGA GAA AGA TGG GGA TTG ACA ACG ATA AAT GTC AAT GAA
 811
AAC TAT AAT AGA CTA ATT AGG CAT ATT GAT GAA TAT GCT GAT CAC TGT GCA AAT ACG TAT AAT CGG GGA TTA AAT AAT TTA CCG AAA TCT
 901
ACG TAT CAA GAT TGG ATA ACA TAT AAT CGA TTA CGG AGA GAC TTA ACA TTG ACT GTA TTA GAT ATC GCC GCT TTC TTT CCA AAC TAT GAC
```

EP 0 295 156 B1

991
AAT AGG AGA TAT CCA ATT CAG CCA GTT GGT CAA CTA ACA AGG GAA GTT TAT ACG GAC CCA TTA ATT AAT TTT AAT CCA CAG TTA CAG TCT

1081
GTA GCT CAA TTA CCT ACT TTT AAC GTT ATG GAG AGC AGC GCA ATT AGA AAT CCT CAT TTA TTT GAT ATA TTG AAT AAT CTT ACA ATC TTT

1171
ACG GAT TGG TTT AGT GTT GGA CGC AAT TTT TAT TGG GGA GGA CAT CGA GTA ATA TCT AGC CTT ATA GGA GGT GGT AAC ATA ACA TCT CCT

1261
ATA TAT GGA AGA GAG GCG AAC CAG GAG CCT CCA AGA TCC TTT ACT TTT AAT GGA CCG GTA TTT AGG ACT TTA TCA ATT CCT ACT TTA CGA

1351
TTA TTA CAG CAA CCT TGC CAG CGC CAC CAT TTT AAT TTA CGT GGT GGT GAA GGA GTA GAA TTT TCT ACA CCT ACA AAT AGC TTT ACG TAT

1441
CGA GGA AGA GGT ACG GTT GAT TCT TTA ACT GAA TTA CCG CCT GAG GAT AAT AGT GTG CCA CCT CGC GAA GGA TAT AGT CAT CGT TTA TGT

1531
CAT GCA ACT TTT GTT CAA AGA TCT GGA ACA CCT TTT TTA ACA ACT GGT GTA GTA TTT TCT TGG ACG CAT CGT AGT GCA ACT CTT ACA AAT

1621
ACA ATT GAT CCA GAG AGA ATT AAT CAA ATA CCT TTA GTG AAA GGA TTT AGA GTT TGG GGG GGC ACC TCT GTC ATT ACA GGA CCA GGA TTT

1711
ACA GGA GGG GAT ATC CTT CGA AGA AAT ACC TTT GGT GAT TTT GTA TCT CTA CAA GTC AAT ATT AAT TCA CCA ATT ACC CAA AGA TAC CGT

1801
TTA AGA TTT CGT TAC GCT TCC AGT AGG GAT GCA CGA GTT ATA GTA TTA ACA GGA GCG GCA TCC ACA GGA GTG GGA GGC CAA GTT AGT GTA

```
1981  GAT ATG CCT CTT CAG AAA ACT ATG GAA ATA GGG TGG AAC TTA ACA TCT AGA ACA TTT AGA TAT ACC GAT TTT AGT AAT CCT TTT TCA TTT
2071  AGA CCT AAT CCA GAT ATA ATT GGG ATA AGT GAA CAA CCT CTA TTT GGT GCA GGT TCT ATT AGT AGC GGT GAA CTT TAT ATA GAT AAA ATT
2161  GAA ATT ATT CTA GCA GAT GCA ACA TTT GAA GCA GAA TCT GAT TTA GAA AGA GCA CAA AAG GCG GTG AAT GCC CTG TTT ACT TCT CCC AAT
2251  CAA ATC GGG TTA AAA ACC GAT GTG ACG GAT TAT CAT ATT GAT CAA GTA TCC AAT TTA TCA GAT GAA TTT TGT TTA CTG GAT
2341  GAA AAG CTA GAA TTG TCC GAG AAA GTC AAA CAT CGC AAG CTC AGT GAT GAG CGG AAT TTA CTT CAA GAT GAG CGG AGA GGG ATC
2431  AAT AGA CAA CCA GAC CGT GGC TGG AGA GGA GAT AAT ACC ATC CAA GGA GAT GAG TAC GTC ACA CTA
2521  CCG GGT ACC GTT GAT GAG TGC TAT CCT ACG TAT TTA TAT CAG AAA ATA GAT GAG TCG CGT TAT ACC CGT TAT GAA TTA AGA
2611  GGG TAT ATC ATC GAA GAT AGT CAA GAC TTA GAA ATC TAT TTG ATC GCG TAC AAT GCA AAA CAC GAA ATA GTG GCA GGC ACG GGT TCC
2701  TTA TGG CCG CTT TCA GCC CAA AGT CCA ATC GGA AAG TGT GGA GAA CCG AAT CGA TGC GCG CCA CAC CTT GAA TGG AAT CCT CTA GAT
      TGT TCC TGC AG
```

De façon particulière, des séquences de nucléotides de l'invention codant pour un polypeptide toxique de manière spécifique vis-à-vis des larves de lépidoptères de la famille des Noctuelles, de préférence vis-à-vis de S.littoralis comprennent ou sont constituées par l'enchaînement (III) défini précédemment.

L'enchaînement (III), compris dans la séquence de nucléotides de l'invention, contient 2711 nucléotides. Ce fragment comprend notamment le promoteur potentiel du gène de la δ-endotoxine active sur S.littoralis.

Entrent naturellement dans le cadre de la présente invention, des séquences de nucléotides modifiées par rapport aux enchaînements (I) ou (III) décrits ci-dessus, dans la mesure où ces modifications ne génèrent pas des variations sensibles de la toxicité du polypeptide codé par la séquence modifiée, vis-à-vis de S.littoralis.

Ces modifications peuvent par exemple consister en des délétions, substitutions, recombinaisons.

Ainsi les séquences de nucléotides (I) et (III) comportent en leur position 611 un nucléotide variable correspondant l'adénine (A) dans la séquence (I) et à la cytosine (C) dans la séquence (III). Ces nucléotides entrent dans la composition des codons respectifs GAA et GCA qui codent respectivement pour les acides aminés acide glutamique (GLU) et alanine (ALA) dans les séquences respectives II et IV.

De même toute séquence de nucléotides hybridable avec celle des enchaînements (I) ou (III), telle qu'obtenue par transformation enzymatique inverse de l'ARN correspondant ou encore par synthèse chimique entre également dans le cadre des définitions de l'invention.

La séquence de nucléotides de formule (III) commence par un codon d'initiation ATG situé en position 241 et qui représente le début d'une phase ouverte de lecture de 2470 nucléotides.

L'invention concerne en outre une séquence de nucléotides caractérisée en ce qu'elle code pour un polypeptide comprenant la séquence (IV) d'acides aminés ci-après :

MET GLU GLU ASN ASN GLN ASN GLN CYS ILE

271
PRO TYR ASN CYS LEU SER ASN PRO GLU GLU VAL LEU LEU ASP GLY GLU ARG ILE SER THR GLY ASN SER SER ILE ASP ILE SER LEU SER

361
LEU VAL GLN PHE LEU VAL SER ASN PHE VAL PRO GLY GLY GLY PHE LEU VAL GLY LEU ILE ASP PHE VAL TRP GLY ILE VAL GLY PRO SER

451
GLN TRP ASP ALA PHE LEU VAL GLN ILE GLU GLN LEU ILE ASN GLU ARG ILE ALA GLU PHE ALA ARG ASN ALA ALA ILE ALA ASN LEU GLU

541
GLY LEU GLY ASN ASN PHE ASN ILE TYR VAL GLU ALA PHE LYS GLU TRP GLU GLU ASP PRO ASN ASN PRO ALA THR ARG THR ARG VAL ILE

631
ASP ARG PHE ARG ILE LEU ASP GLY LEU LEU GLU ARG ASP ILE PRO SER PHE ARG ILE SER GLY PHE GLU VAL PRO LEU LEU SER VAL TYR

721
ALA GLN ALA ALA ASN LEU HIS LEU ALA ILE LEU ARG ASP SER VAL ILE PHE GLY GLU ARG TRP GLY LEU THR THR ILE ASN VAL ASN GLU

811
GLU TYR ASN ARG LEU ILE ARG HIS ILE ASP GLU TYR ALA ASP HIS CYS ALA ASN THR TYR ASN ARG GLY LEU ASN ASN LEU PRO LYS SER

901
THR TYR GLN ASP TRP ILE THR TYR ASN ARG LEU ARG ARG ASP LEU THR LEU THR VAL LEU ASP ILE ALA ALA PHE PHE PRO ASN TYR ASP

EP 0 295 156 B1

EP 0 295 156 B1

991

ASN ARG ARG TYR PRO ILE GLN PRO VAL GLY GLN LEU THR ARG GLU VAL TYR THR ASP PRO LEU ILE ASN PHE ASN PRO GLN LEU GLN SER

1081
VAL ALA GLN LEU PRO THR PHE ASN VAL MET GLU SER SER ALA ILE ARG ASN PRO HIS LEU PHE ASP ILE LEU ASN ASN LEU THR ILE PHE

1171
THR ASP TRP PHE SER VAL GLY ARG ASN PHE TYR TRP GLY GLY HIS ARG VAL ILE SER SER LEU ILE GLY GLY GLY ASN ILE THR SER PRO

1261
ILE TYR GLY ARG GLU ALA ASN GLN GLU PRO PRO ARG SER PHE THR PHE ASN GLY PRO VAL PHE ARG THR LEU SER ILE PRO THR LEU ARG

1351
LEU LEU GLN GLN PRO CYS GLN ARG HIS HIS PHE ASN LEU ARG GLY GLY GLU GLY VAL GLU PHE SER THR PRO THR ASN SER PHE THR TYR

1441
ARG GLY ARG GLY THR VAL ASP SER LEU THR GLU LEU PRO PRO GLU ASP ASN SER VAL PRO PRO ARG GLU GLY TYR SER HIS ARG LEU CYS

1531
HIS ALA THR PHE VAL GLN ARG SER GLY THR PRO PHE LEU THR THR GLY VAL VAL PHE SER TRP THR HIS ARG SER ALA THR LEU THR ASN

1621
THR ILE ASP PRO GLU ARG ILE ASN GLN ILE PRO LEU VAL LYS GLY PHE ARG VAL TRP GLY GLY THR SER VAL ILE THR GLY PRO GLY PHE

1711
THR GLY GLY ASP ILE LEU ARG ARG ASN THR PHE GLY ASP PHE VAL SER LEU GLN VAL ASN ILE ASN SER PRO ILE THR GLN ARG TYR ARG

1801
LEU ARG PHE ARG TYR ALA SER SER ARG ASP ALA ARG VAL ILE VAL LEU THR GLY ALA ALA SER THR GLY VAL GLY GLY GLN VAL SER VAL

```
1151 GLN MET PRO LEU GLN LYS THR MET GLU ILE GLY GLY.GLU ASN ILE GLY ASN LEU THR SER ARG THR PHE ARG TYR THR ASP PHE SER.ASN PRO PHE SER PHE

1501 ARG ALA ASN PRO ASP ILE ILE GLY ILE SER GLU GLN GLN PRO LEU PHE GLY ALA GLY SER ILE SER GLU LEU TYR ILE ASP LYS ILE

2071 GLU ILE ILE LEU ALA ASP ALA THR PHE GLU ALA.GLU ARG ALA GLN LYS ALA GLU VAL ASN ALA LEU PHE SER SER ASN

2161 GLN ILE GLY LEU LYS THR ASP VAL THR ASP TYR.HIS ILE ASP GLN VAL SER ASN LEU VAL ASP CYS LEU SER ASP GLU PHE CYS LEU ASP

2251 GLU LYS ARG GLU LEU SER GLU LYS VAL LYS HIS ALA LYS LEU ARG LEU SER ASP.GLU LEU LEU GLN ASP PRO ASN PHE ARG GLY ILE

2341 ASN ARG GLN PRO ASP ARG GLY TRP ARG GLY SER THR ILE GLN GLY ASP ASP VAL PHE LYS GLU ASN TYR VAL THR LEU

2431 PRO GLY THR VAL ASP GLU CYS TYR PRO THR TYR LEU TYR GLN LYS ILE ILE ASP GLU SER LYS LEU ALA TYR THR ARG TYR GLU LEU ARG

2521 GLY TYR ILE GLU ASP SER GLN ASP LEU GLU ILE GLU ILE TYR LEU ILE ALA TYR ASN ALA LYS HIS GLU ILE VAL ASN VAL PRO GLY THR GLY SER

2611 LEU TRP PRO LEU SER PRO ALA GLN SER PRO ILE GLY LYS CYS GLY GLU PRO ASN ARG CYS ALA PRO HIS LEU GLU TRP ASN PRO ASP LEU ASP

2701 CYS SER CYS
```

L'invention se rapporte également aux vecteurs recombinants d'expression et de clonage comportant plus particulièrement au moins une séquence de nucléotides telle que définie ci-dessus, en particulier au moins une partie de la séquence d'environ 3kb.

Un vecteur recombinant particulier est par exemple un plasmide comprenant le fragment HindIII-PstI de la séquence de nucléotides de l'invention, inséré dans un vecteur pUC9. Un premier vecteur préféré est le plasmide pHT71 dont la construction est rapportée dans les ensembles ci-après, qui comprend un fragment

EP 0 295 156 B1

d'ADN HindIII-PstI selon l'invention constitué uniquement d'ADN provenant de la souche aizawai 7-29.

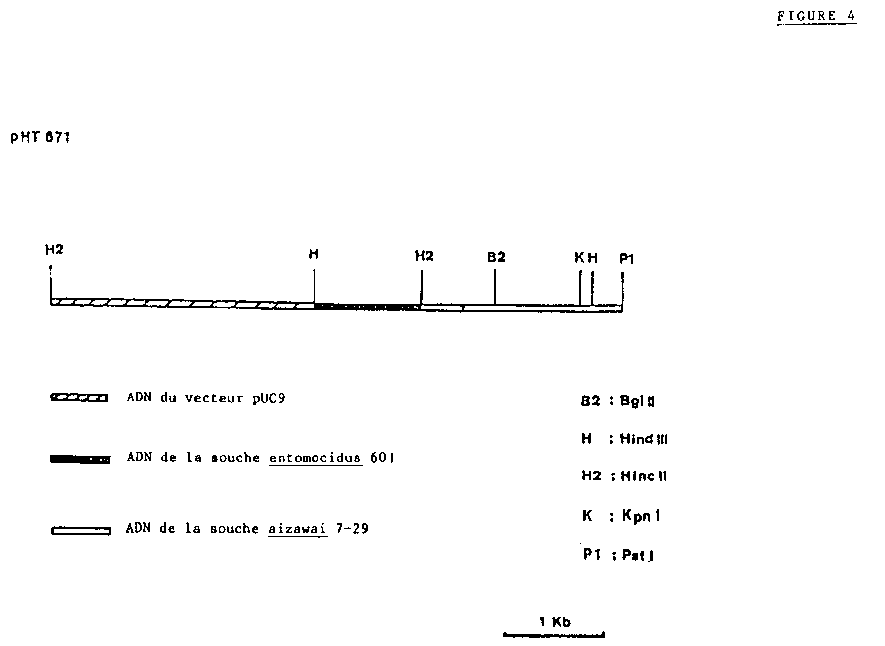

Un autre vecteur recombinant est constitué par le plasmide pHT 671 dont la construction est donnée dans la figure 4. Ce plasmide comprend un fragment HindIII-PstI chimère, obtenu en fusionnant un fragment d'ADN HindIII-HincII de 1,1 kb provenant de la souche entomocidus 6-01 avec un fragment HincII-PstI de 1,9 kb issu de la souche aizawai 7-29.

Entrent également dans le cadre de l'invention les souches bactériennes modifiées qui comportent l'une des séquences nucléotides définies ci-dessus ou encore un vecteur recombinant d'expression et de clonage défini précédemment, de préférence le plasmide pHT 671 ou le plasmide pHT71.

L'invention vise en outre un polypeptide toxique vis-à-vis des larves de lépidoptères et de manière préférentielle vis-à-vis de S.littoralis, s'attaquant aux feuilles de coton ou des autres cultures telles qu'énumérées ci-dessus, caractérisé en ce qu'il est capable de former un complexe immunologique avec des anticorps dirigés contre des polypeptides à activité larvicide vis-à-vis de S.littoralis.

L'invention vise plus particulièrement la partie NH$_2$-terminale de ce polypeptide qui renferme l'activité larvicide.

L'extrémité de la partie NH$_2$-terminale active répond à la séquence (II) d'acides aminés donnée ci-dessus.

Un polypeptide préféré de l'invention est celui qui répond à cette séquence (II) et répond à la séquence (IV) d'acides aminés donnée dans les pages précédentes. Ce polypeptide répondant à la séquence (IV) comprend 823 acides aminés. Sa masse moléculaire calculée est de 92906 Da.

Cette séquence de δ-endotoxine a été comparée aux séquences en acides aminés des δ-endotoxines provenant d'autres souches de B.thuringiensis actives sur les lépidoptères et dont les gènes ont été isolés et séquencés précédemment : il s agit des δ-endotoxines des souches kurstaki HD1 (19), kurstaki HD73 (20), berliner 1715 (21) et (22) Sotto (23) et aizawai IPL7 (24).

Les résultats de ces comparaisons indiquent que toutes sont différentes dans le second quart de la molécule (acides aminés 281 à 620) alors que la partie NH$_2$ terminale (acides aminés 1 à 280) et le domaine COOH terminal (acides aminés 621 à 1175) de la protéine sont très conservés et ne diffèrent que par quelques acides aminés. En revanche la δ-endotoxine correspondant à la séquence (IV) ci-dessus présente des différences importantes avec les autres δ-endotoxines, aussi bien dans la partie NH$_2$ terminale (acides aminés 1 à 280) que dans le second quart de la molécule (acides aminés 281 à 620). Les résultats de ces comparaisons indiquent encore que la moitié NH$_2$-terminale de la molécule (acides aminés 1 à 620) qui correspond à la fraction toxique de la protéine ne présente que 46% d homologie avec les autres δ-endotoxines. Les différences les plus importantes se situent dans la seconde moitié de la partie toxique de la molécule (acides aminés 281 à 620) avec seulement 36% d'acides aminés identiques, la partie NH$_2$-terminale (acides aminés 1 à 280) présentant quant à elle 58% d'acides aminés identiques avec les autres δ-endotoxines. De telles différences importantes n'ont jamais été observées jusqu'à présent dans la partie NH$_2$-terminale de la fraction toxique de la molécule, parmi les δ-endotoxines actives sur les lépidoptères.

Pour obtenir une séquence de nucléotides entrant dans le cadre de l'invention, codant pour au moins la partie active d'un polypeptide présentant une toxicité spécifique vis-à-vis de larves de lépidoptères de la famille des Noctuelles, de préférence vis-à-vis de S.littoralis, on a recours, conformément à l'invention, aux étapes suivantes, à savoir :

- la réalisation d'une hybridation moléculaire entre d'une part une séquence de nucléotides d'une souche de B.thuringiensis active contre S.littoralis, et d'autre part au moins deux séquences de nucléotides, utilisées comme sondes, provenant de la partie 5′ d'un fragment de restriction d'un gène de δ-endotoxine de B.thuringiensis, cette partie codant pour la partie NH$_2$-terminale du polypeptide actif sur les larves de lépidoptères et de la partie 3′ de ce fragment codant pour la partie COOH du polypeptide,
- l'isolement du fragment hybridé,
- son clonage dans un vecteur, suivi de sa purification.

De manière avantageuse, les sondes d'hybridation utilisées sont obtenues à partir d'un gène de δ-endotoxine provenant de la souche aizawai 7-29 codant pour une protéine de 130 kDa, active contre P.brassicae et inactive vis-à-vis de S.littoralis, ce gène ayant été cloné dans le plasmide recombinant pHTA2.

Dans un mode de réalisation du procédé précédent, le fragment recombiné au vecteur dans l'étape de clonage est élaboré à partir d'un fragment de restriction HindIII-PstI provenant d'une unique souche de B.thuringiensis, de préférence aizawai 7-29. En particulier ce fragment est porté préférentiellement par le plasmide recombinant pHTA6 tel qu'isolé à l'aide d'une sonde constituée par un fragment PvuII de 2kb du plasmide pBT15-88 correspondant à la partie interne d'un gène du cristal chromosomique de la souche berliner 1715, à partir de clones transformants renfermant des séquences nucléotides issues de souches B.thuringiensis actives vis-à-vis de larves de lépidoptères inter-alia de S.littoralis.

Dans un autre mode de réalisation de l'invention, le fragment recombiné au vecteur dans l'étape de clonage est élaboré à partir de plusieurs séquences de nucléotides issues de vecteurs recombinants contenant des séquences de nucléotides d'au moins deux souches différentes de B.thuringiensis, possédant les mêmes cartes de restriction et contenant elles-mêmes tout ou partie des séquences de nucléotides capables de coder pour un polypeptide actif, de manière préférentielle, vis-à-vis de S.littoralis.

Dans ce cas, le fragment recombiné utilisé dans l'étape de clonage est un fragment de 3kb environ, élaboré avantageusement à partir d'un fragment de restriction HindIII-HincII d'environ 1,1kb provenant de la souche entomocidus 6-01 et d'un fragment HincII-PstI d'environ 1,9kb de la souche aizawai 7-29. Il correspond à un gène tronqué de δ-endotoxine.

Les fragments de restriction HindIII-HincII et HincII-PstI sont portés plus spécialement par les plasmides recombinants respectifs pHTE6 et pHTA6 tels qu'isolés à l'aide de la sonde constituée par le fragment PvuII dont question ci-dessus.

L'étude de la toxicité, vis-à-vis des larves de lépidoptères, des souches bactériennes modifiées à l'aide des séquences de nucléotides définies ci-dessus, a permis de mettre en évidence leur activité toxique élevée, notamment à l'égard des chenilles de S.littoralis.

Cette activité a été appréciée au regard de l'indice de spécificité correspondant au rapport

$$\frac{\text{CL50 } \underline{\text{S.littoralis}}}{\text{CL50 } \underline{\text{P.brassicae}}}$$

dans lequel "CL50" représente la concentration léthale tuant 50% des larves en 72 heures.

L'utilisation d'un tel indice permet d'évaluer l'activité des souches bactériennes étudiées sans avoir à considérer le taux d'expression des polypeptides.

Les résultats obtenus, qui sont rapportés dans les exemples qui suivent, et les valeurs de DL 50 qui en sont déduites, ont montré que les souches bactériennes modifiées selon l'invention présentent une activité toxique vis-à-vis de S.littoralis plus spécifique que les protéines de cristal natives des souches azawai 7-29 ou berliner 1715.

L'invention vise donc l'application, pour l'élaboration de compositions larvicides toxiques de préférence vis-à-vis de S.littoralis, de ces souches modifiées, de vecteurs recombinants renfermant les séquences nucléotidiques définies plus haut, en particulier du plasmide pHT671 et du plasmide pHT71, et de ces séquences elles-mêmes.

Les compositions larvicides de l'invention sont alors caractérisées en ce qu'elles renferment une quantité efficace de polypeptides tels que définis ci-dessus ou exprimés par les séquences nucléotidiques évoquées plus haut.

pour produire ces polypeptides on met avantageusement en oeuvre les gènes tronqués de δ-endotoxine correspondant aux séquences nucléotidiques de l'invention.

Ces gènes peuvent être utilisés pour produire en excès le polypeptide toxique dans des microorganismes permettant l'expression des vecteurs recombinants ci-dessus. Des souches de microorganismes appropriées comprennent E.coli ou encore B.subtilis.

Ces gènes tronqués peuvent être réintroduits dans les souches de B.thuringiensis ou dans des espèces apparentées telle que B.cereus, selon les techniques classiques, par exemple, par transformation, conjugaison ou transduction. Ces techniques permettent de produire le polypeptide toxique en grande quantité sans avoir à modifier au préalable la région naturelle du promoteur des gènes de δ-endotoxine de B.thuringiensis.

Cette transformation peut être effectuée en utilisant des méthodes dérivées de la transformation des protoplastes de B.subtilis selon (11) ou des cellules végétatives de B.thuringiensis comme décrit dans (12).

L'introduction de plasmides recombinants par un système de type conjugaison, peut être réalisée en utilisant B.thuringiensis comme souche hôte et B.subtilis ou Streptococcus faecalis comme souches de type donneur, en opérant seion (13) et (14).

En variante, les séquences de nucléotides sont introduites dans des microorganismes vivant dans l'environnement ou en association avec les plantes et capables d exprimer des vecteurs recombinants renfermant ces séquences. L'introduction peut être effectuée dans des micoorganismes teis que Pseudomonas en opérant selon le procédé décrit dans (17), par l'intermédiaire de vecteurs plasmidiques contenant le transposon Tn5 et le gène de la toxine, ou Azospirillum ou Rhizobium par l'intermédiaire de vecteurs suicides dérivés du plasmide RP4 et de plasmides mobilisateurs fonctionnels chez les bactéries gram négatives (pRK2013 par exemple) selon les procédés décrits dans (18).

Les gènes tronqués sont seuls dans les souches de Bacilli, ou en variante sont associés à différents gènes de δ-endotoxine ce qui permet d'obtenir des cristaux synthétisés par ces souches spécifiquement toxiques, vis-à-vis d'espèces données de Noctuelles, ou toxiques à la fois vis-à-vis des Noctuelles et d'insectes sensibles aux autres δ-endotoxines. Ces recombinaisons, effectuées in vitro ou in vivo avec les séquences nucléotidiques de l'invention et d'autres gènes de δ-endotoxines présentant des spécificités toxiques différentes, conduisent à la construction de nouveaux gènes codant pour de nouvelles protéines toxiques hybrides présentant un large spectre activité vis-à-vis des insectes. Ces nouveaux gènes et ces nouvelles protéines entrent également dans le cadre de l'invention.

Dans ces applications, les séquences nucléotidiques de l'invention peuvent être transférées et exprimées dans des plantes sensibles à S.littoralis afin de diminuer les ravages causés par ces insectes.

Parmi les plantes à protéger, on citera : le coton, le trèfle, la tomate et la luzerne.

Le transfert du gène tronqué dans des plants de coton, peut être réalisé par transformation mettant en jeu des souches telles que Agrobacterium comme décrit dans (15).

L'invention concerne en outre les cellules végétales, les plantes et les graines renfermant les séquences nucléotidiques definies ci-dessus.

Les cellules végétales selon l'invention sont des cellules dont le génome, après transformation par un procédénon essentiellement biologique, possède de façon stable une séquence de nucléotides capable d'exprimer un polypeptide toxique vis-à-vis de S.littoralis, telle que définie ci-dessus. L'invention concerne également les cellules végétales issues de leur division.

Les plantes selon l'invention sont des plantes transformées par un procédé non essentiellement biologique, ayant en particulier pour prédateur S.littoralis, dont le génome possède de façon stable une séquence de nucléotides telle que définie ci-dessus, capable d'exprimer un polypeptide toxique vis-à-vis de S.littoralis. Il s'agit aussi de plantes issues de leur reproduction, de leur multiplication ou de croisements hybrides.

Selon un autre aspect, l'invention concerne des plantes ayant en particulier pour prédateur S.littoralis, possédant en plus de leurs caractères génotypiques et phénotypiques initiaux la propriété d'exprimer un polypeptide toxique de manière préférentielle vis-à-vis de S.littoralis, cette propriété résultant de l'insertion dans leur génome par manipulation génétique d'une séquence de nucleotides capable d'exprimer ledit polypeptide.

L'invention vise en outre des graines capables de donner une plante telle que définie ci-dessus ou issues d'une telle plante, caractérisées en ce qu'elles ont intégré dans leur génome, par manipulation génétique une séquence de nucléotide décrite plus haut.

D'autres caractéristiques et avantages de l'invention apparaîtront dans la suite de la description et en se reportant aux exemples dans lesquels :
- la figure 1 représente la carte de restriction des plasmides pHTA6 et pHTE6,
- la figure 2, la carte de restriction d'un gène d'une protéine de cristal de la souche aizawai 7.29 cloné dans le plasmide pHTA2 et définissant les fragments d'ADN qui sont utilisés comme sonde,
- la figure 3, présente le fragment de 6,6kb cloné dans pHTA6 et le résultat d'une hybridation effectuée entre ce fragment et les sondes décrites dans la figure 2,
- la figure 4, la carte de restriction du plasmide pHT671, et
- la figure 5, les photographies de tests d'immunodiffusion.

Les expériences d'hybridation réalisées pour la mise en oeuvre de l'invention ont été effectuées à 42°C pendant 24 h. dans une solution contenant 5 x SSC, 30% de formamide et 1 Denhardt (7) en présence de la sonde ADN marquée au $^{32}$P. Les filtres sont lavés à 42°C, 20 mn, en utilisant successivement les solutions suivantes : 5 x SSC dans 30% de formamide, 5 x SSC, 2 x SSC, 1 x SSC et 0,5 x SCC avant séchage à température ambiante.

EXEMPLE I - Construction d'une séquence d'ADN de 3kb environ contenant un gène chimère d'une toxine insecticide

Cette construction comprend :
1/ la préparation de banques de gènes de B.thuringiensis
2/ la sélection et la caractérisation des clones transformants renfermant les gènes d'une protéine du cristal et des séquences nucléotidiques responsables de l'activité larvicide,
3/ recombinaison in vitro de ces séquences dans un vecteur de clonage avec construction du plasmide pHT671.

Ces différentes étapes sont réalisées comme suit :

1/ Préparation de banques de gènes de B. thuringiensis.

L'ADN total des souches aizawai 7-29 et entomocidus 6-01 de Bacillus thuringiensis est purifié en utilisant la méthode rapportée dans (1) et 50μg de chaque ADN purifié sont complètement digérés avec l'enzyme de restriction PstI.

L'ADN digéré par PstI est ananlysé par électrophorèse horizontale sur gel d'agarose à 0,8% et des fragments d'ADN d'une taille de 5 à 8 kb sont récupérés à partir des gels d'agarose, par éléctroélution, de la manière décrite dans (2).

Les fragments d'ADN purifiés de 5-8 kb de la souche aizawai 7-29 sont ligaturés à l'ADN du vecteur de clonage pUC18 digéré par PstI selon (3).

Les fragments d'ADN purifiés de 5-8 kb de la souche entomocidus 6-01 sont ligaturés à l'ADN du vecteur de clonage pUC9 digéré par PstI. Les cellules de E.coli JM83 sont transformées avec le mélange de ligature comme décrit dans (4).

Les clones transfomants de E.coli sont sélectionnés sur milieu LB contenant 100 μg/ml d'ampicilline.

2/ Isolement et caractérisation des clones transformants contenant les gènes d'une protéine de cristal.

A/ Criblage des cellules de E.coli transformées à l'aide d'un fragment interne d'un gène de la protéine du cristal marqué au $^{32}$p, utilisé comme sonde :

Des clones transformants contenant des plasmides recombinants portant le gène du cristal sont détectés par hybridation sur colonies, suivant la méthode décrite dans (5), utilisant comme sonde un fragment PvuII de 2 kb du plamide pBT 15-88 (EMBO J., 1 : 791-799) correspondant à une partie interne du gène de la protéine du cristal, situé sur le chromosome de la souche berliner 1715.

B/ Caractérisation des plasmides recombinants présents dans les clones qui réagissent avec la sonde ci-dessus.

Deux plasmides recombinants, pHTA6 et pHTE6, isolés respectivement des banques de gènes construites à partir des souches aizawai 7-29 et entomocidus 6-01, présentent une homologie avec cette sonde. Dans chaque cas, un fragment d'ADN d'environ 6,6 kb a été cloné.

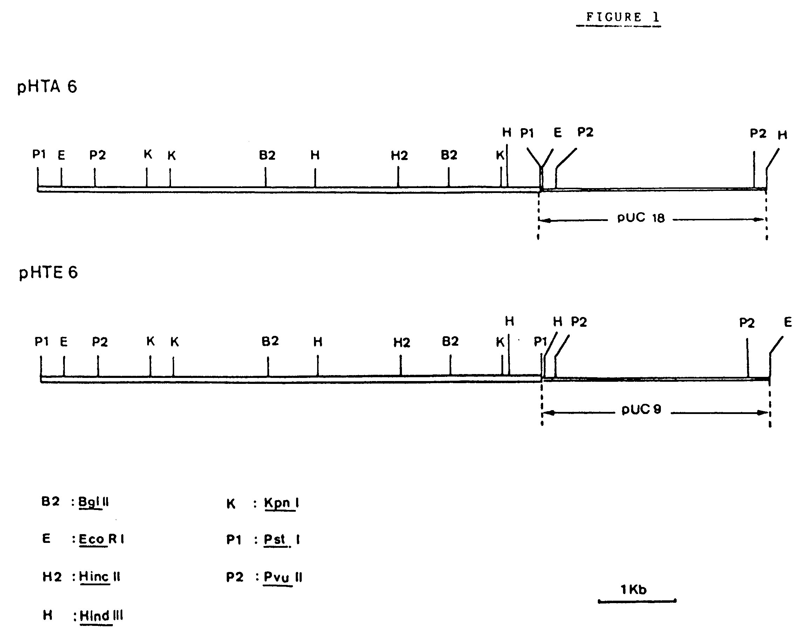

La carte de restriction des deux plasmides est donnée sur la figure 1. La comparaison des sites de restriction montre que les deux fragments d'ADN clonés semblent identiques.

Afin de délimiter les séquences correspondant au gène de la δ-endotoxine, différents fragments d'ADN marqués au $^{32}$P, provenant d'un gène du cristal précédemment caractérisé, et cloné dans le plasmide recombinant pHTA2, sont utilisés comme sondes. Ce dernier gène du cristal également originaire de la souche aizawai 7-29 code pour une protéine de 130 kd active contre P.brassicae mais pas contre S.littoralis. Ce type de gène possède la même carte de restriction que le gène de la δ-endotoxine issu de la souche berliner 1715. Sur la figure 2, on a rapporté la carte de restriction de ce gène de la protéine du cristal de la souche de aizawai 7.29 cloné dans le plasmide pHTA2. Les traits épais indiqués au-dessus de la carte correspondent aux fragments utilisés comme sondes d'hybridation.

Les plasmides pHTA6 et pHTE6 sont hydrolysés par différentes endonucléases de restriction, analysés par électrophorèse horizontale sur gel d'agarose à 0,8% et hybridés avec les différentes sondes.

Le transfert de l'ADN sur des filtres de nitrocellulose est réalisé suivant la méthode de SOUTHERN décrite dans (6). L'hybridation est conduite à 42°C pendant 24 heures dans une solution contenant : 5X SSC, 30% de formamide et un mélange 1X Denhardt décrit dans (7) en présence d'une sonde d'ADN marquée au $^{32}$P. Les filtres sont ensuite lavés à 42°C pendant 20 minutes, en utilisant successivement les solutions suivantes : 5 SSC dans 50% de formamide, 5 SSC, 2 SSC, 1 SSC et 0,5 SSC avant d'être séchés à la température ambiante.

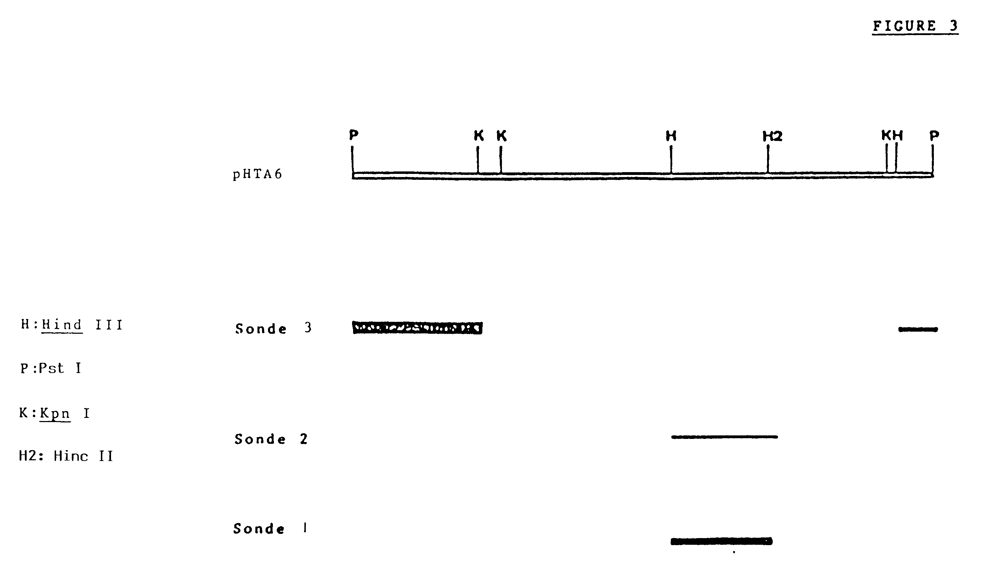

Les résultats de ces expériences d'hybridation sont résumés sur la figure 3. Il apparaît que chaque extrémité des fragments d'ADN de 6,6 kb clonés présente une homologie avec les sondes. Le fragment PstI-KpnI de 1,5 kb réagissant avec la sonde n° 3 correspond à l'extrémité 3′ d'un gène de la protéine du cristal présent à la fois dans les souches aizawai 7-29 et entomocidus 6-01. Comme il est indiqué sur la figure 3, les sondes n° 1 et n° 2 correspondant à l'extrémité 5′ du gène de la δ-endotoxine de pHTA2, s'hybrident avec le fragment HindIII-HincII de 1,1 kb contenu dans le plasmide pHTA6. La sonde n° 3 qui couvre l'extrémité 3′ du gène de la δ-endotoxine de pHTA2, s'hybride avec le fragment HindIII-PstI de 0,4kb contenu dans le plasmide pHTA6. Il doit être noté qu'un faible signal d'hybridation est obtenu avec la sonde n° 2 alors que les deux autres sondes donnent des signaux facilement détectables.

A partir de ces résultats, les inventeurs ont établi que le fragment d'ADN HindIII-PstI de 3 kb correspond à une grande partie d'un gène de la δ-endotoxine qui commence près du site HindIII central. Il apparaît clairement au vu des résultats des expériences d'hybridation que le gène de la δ-endotoxine présente des différences substantielles avec ceux caractérisés dans l'art antérieur. Sur la base de ces résultats il a été décidé de cloner le fragment de 3 kb HindIII-PstI dans le vecteur pUC9.

3/ Construction du plasmide pHT 671 contenant un gène chimère de la toxine insecticide reconstitué.

Le fragment d'ADN HindIII-HincII de 1,1 kb issu du plasmide pHTE6 et le fragment d'ADN HincII-PstI de 1,9kb issu du plasmide pHTA6 sont purifiés sur gels d'agarose.

Des quantités égales des deux fragments d'ADN purifiés et de l'ADN de pUC9 digéré avec HindIII et PstI sont mélangées et ligaturées. Le mélange de ligature est utilisé pour transformer des cellules compétentes de E.coli JM83, puis les cellules transformées de E.coli sont selectionnées sur milieu LB contenant de l'ampicilline. L'un des clones recombinants interessant examiné contient un plasmide désigné par pHT671, dont la carte de restriction a été déterminée et est représentée sur la figure 4. Ce plasmide (pHT671) contient un fragment d'ADN de 3 kb inséré dans le vecteur pUC9. Cette séquence d'ADN a la même carte de restriction que les fragments HindIII-PstI de 3 kb contenus dans les plasmides pHTA6 et pHTE6, mais correspond à une molécule d'ADN reconstituée construite par recombinaison in vitro à partir de séquences d'ADN provenant des souches aizawai 7-29 d'une part et entomocidus 6-01 d'autre part.

EXEMPLE II : Etude de la séquence nucléotidique de la région du promoteur et de la région codant pour la partie NH$_2$ terminale de la $\delta$-endotoxine active contre les Noctuidae.

Le fragment HindIII-HincII de pHT671 est séquencé à conformément la méthode décrite dans (8) en utilisant un système M13. Pour obtenir des fragments d'ADN clonés se chevauchant partiellement qui seront utilisés dans le séquençage de l'ADN, on a recours à la méthode de sous-clonage par délétion dans M13, développée par DALE et al (9).

La séquence de 940 nucléotides du fragment HindIII-HincII qui est d'une longueur d'environ 1 kilobase correspond à l'enchaînement I ci-dessus.

L'analyse de cette séquence montre que la plus grande phase ouverte de lecture commence à la position 241 et qu'un site potentiel de liaison aux ribosomes GGAGG se trouve six paires de base en amont de ce codon ATG (position 230 à 235). La région localisée entre les nucléotides 137 et 177 (position -103 à -63 en amont du codon ATG) est fortement homologue à la région présente en amont du gène du cristal de la souche kurstaki HD1 Dipel (BTK) séquencée par WONG et al (1983) et décrite dans (16) et dont les auteurs ont montré qu'elle contient trois promoteurs BtI, BtII, et Ec fonctionnels dans B.thuringiensis et E.coli respectivement. La comparaison entre les séquences en acides aminés déduites des 750 premiers nucléotides des gènes de BTK et pHT671, montre que ces polypeptides présentent des différences significatives au niveau de la moitié N-terminale de la partie active issue de la protoxine (seulement 66% d'homologie stricte). Il est important de noter que c'est la première fois qu'un gène de la $\delta$-endotoxine isolé à partir d'une souche active contre les lépidoptères code pour un polypeptide qui présente des différences substantielles dans cette région. En effet, ce domaine N-terminal apparaît fortement conservé (plus de 97% d'homologie stricte) parmi tous les gènes du cristal actifs sur lépidoptères qui ont été séquencés jusqu'à présent. Par ailleurs, les inventeurs ont montré que le taux de variabilité est du même ordre si l'on considère les séquences nucléotidiques de pHT671 et des autres gènes de type lépidoptères.

EXEMPLE III : Construction d'une séquence d'ADN de 2,7kb environ contenant un gène d'une toxine larvicide.

Pour réaliser cette construction on a utilisé l'ADN de la souche aizawai 7.29 de B.thuringiensis jusqu'à l'étape de réalisation du plasmide pHTA6 telle que décrite dans l'exemple I.

Le fragment HindIII-PstI d'environ 2,7kb obtenu à partir du plasmide pHTA6 a ensuite été sous-cloné dans le vecteur pUC9, préalablement hydrolysé par les enzymes de restriction HindIII-PstI, pour donner le plasmide pHT71.

EXEMPLE IV : Etude de la séquence de nucléotides constituant le plasmide pHT71 codant pour un polypeptide toxique vis-à-vis des larves de lépidoptères de la famille des Noctuelles.

Le fragment HindIII-Pst-I de 2,7kb de pHTA6, qui a été sous-cloné dans pHT71, a été séquencé par la technique de Sanger et al.(8) en utilisant le phage M13mp19 et le système de sous-clonage par délétions développé par Dale et al(9). Ce système permet d'obtenir des phages M13 contenant une série de fragments d'ADN se chevauchant partiellement et qui peuvent être utilisés pour le séquençage de l'ADN.

La séquence de nucléotides de ce fragment de 2,7kb qui correspond à l'enchaînement (III) donné ci-dessus, a été déterminée sur les 2 brins d'ADN, exception faite des 212 derniers nucléotides (position 2500 à 2711) qui n'ont été séquencés que sur 1 seul brin.

La séquence nucléotidique de ce fragment Hind-III-PstI a une longueur de 2711 nucléotides. Ce fragment contient le promoteur potentiel ainsi que la plus grande partie du gène de la $\delta$-endotoxine active sur S.littoralis.

EXEMPLE V : Etude de la toxicité spécifique des clones recombinants de E.coli JM83 (pHT671) et JM83 (pHT71) contre S.littoralis.

La toxicité des clones recombinants de E.coli JM83 contenant pHT671 et de E.coli JM83 contenant pHT71 a été déterminée par des tests biologiques sur des chenilles de l'espèce P.brassicae et S.littoralis comme décrit par LECADET et MARTOURET dans (10). Les résultats ont été comparés avec la toxicité spécifique des protéines de cristal natives et purifiées à partir des souches berliner 1715 et aizawai 7-29 entomocidus 6.01 B cereus 569 (contenant le plasmide pBT45, pAM$\beta$1) contre les deux espèces d'insectes. La toxicité spécifique du clone recombinant et des souches de B.thuringiensis est exprimée en termes d' "indice de spécificité" défini précédemment.

Les résultats obtenus sont rapportés dans le tableau 1 ci-après.

Dans ce tableau, pour des souches de E.coli, la concentration 1 correspond à une culture bactérienne de 14 heures concentrée 20 fois, désintégrée par ultrasons ; pour les souches de B.thuringiensis la concentration est exprimée en $\mu$g de protéine cristal par $\mu$l de préparation. L'activité toxique des préparations a été testée par ingestion forcée sur des chenilles au cinquième stade de développement avec 5 $\mu$l de préparation, ou par une technique d'ingestion libre en utilisant des larves au deuxième stade de développement.

TABLEAU 1

| Toxicité comparée d'un clone recombinant et de deux souches de B.thuringiensis vis-à-vis de S.littoralis et P.brassicae. | | | |
|---|---|---|---|
| Souches et plasmides | S.littoralis | | P.brassicae | Indice de spécificité CL50 S.littoralis CL50 P.brassicae |
| | CL50 2ème stade larvaire | CL50 5ème stade larvaire | CL50 5ème stade larvaire | |
| JM83 (pUC18) | > 1 | > 1 | > 1 | - |
| JM83 (pHT671) | 0,04 | 0,13 | 0,72 | 0,2 |
| JM83 (pHTA2) | > 1 | > 1 | 0,03 | > 30 |
| JM83 (pHTA4) | > 1 | > 1 | > 1 | - |
| JM83 (pHT71) | ND | 0,5 | > 1 | < 0.5 |
| berliner 1715 cristaux natifs | ND | 0,11 | 0,007 | 15,7 |
| aizawai 7.29 cristaux natifs | ND | 0,02 | 0,04 | 0,5 |
| entomocidus 601 cristaux natifs | ND | 0,028 | 0,012 | 2,3 |
| B.cereus 569 (pBT45,pAM$\beta$1) | ND | 0,38 | 0,054 | 7 |

L'examen des valeurs de CL50 résumées dans ce tableau 1 montre que les extraits de protéine des clones recombinants JM83 (pHT671) et JM83 (pHT71) sont préférentiellement toxiques contre S.littoralis. En second lieu une comparaison des valeurs de 1 indice de spécificité montre que l'activité larvicide des clones recombinants est plus spécifique à raison de 2,5 fois envers S.littoralis que les protéines du cristal natives de la souche aizawai. Par ailleurs, les clones recombinants de JM83 (pHT671) et JM83 (pHT71) sont très actifs contre un autre insecte de la famille des Noctuidae, Mamestra brassicae (pour le clone JM83 (pHT671) par exemple, la valeur CL50 est de 0,02, en utilisant des larves du deuxième stade de développpement).

Ces deux résultats montrent que le gène de la toxine larvicide construit et cloné dans les plasmides pHT671 et pHT71 code pour une protéine spécifiquement active contre S.littoralis.

D'autres préparations obtenues à partir de clones recombinants contenant des plasmides portant des gènes codant pour d'autres types de δ-endotoxines (pHTA2 et pHTA4) ne sont pas actives sur S.littoralis : on peut voir que le plasmide pHTA2 code pour une δ-endotoxine spécifiquement active sur P.brassicae alors que le plasmide pHTA4 code pour une δ-endotoxine dont l'insecte cible n'a pas encore été identifié. On peut également voir que les inclusions cristallines produites par une souche de Bacillus cereus qui a reçu le plasmide pBT45, un des plasmides de la souche aizawai 7-29 qui porte aussi un gène de δ-endotoxine (le gène d'origine plasmidique de la souche aizawai 7-29), sont également spécifiquement actives sur P.brassicae.

Des résultats similaires sont obtenus en utilisant, à la place des extraits bactériens bruts, des extraits protéiques solubles préparés à partir de différents clones recombinants de E.coli.

Sur la base des valeurs de la CL50 rapportées dans le tableau ci-dessus et d'un poids individuel moyen de 41 mg par larve L5 (cinquième stade larvaire) de S.littoralis. la valeur de la DL50 a été estimée à 2,4μg/gramme de larve pour les cristaux natifs de la souche aizawai 7-29.

Sur ces mêmes bases et sur la base de facteurs d'équivalence permettant de passer de la masse bactérienne totale à la quantité de protéines spécifiques (2% environ des protéines totales chez E.coli JM83 (pHT671), la DL50 correspondant à la toxine produite par l'expression chez E.coli JM83 du gène selon l'invention cloné dans le plasmide pHT671, a été déterminée et estimée à une valeur proche de 5,5 à 6 μg/gramme de larve.

Sur ces mêmes bases et après détermination de la CL50 d'extraits protéiques solubles préparés à partir de cultures broyées de E.coli JM83 (pHT671), la valeur de la DL50 correspondant à la toxine présente dans ces extraits a été estimée à 4,15 μg/gramme de larve.

Dans les deux cas et particulièrement dans le cas des broyats de E.coli, les valeurs de DL50 calculées sont approximatives et supérieures à celle des cristaux natifs, puisqu'il ne s'agit pas de toxine purifiée. Cependant ces données indiquent sans ambiguité que le gène exprimé par pHT671 détermine une δ-endotoxine présentant la spécificité vis-à-vis de S.littoralis. En effet, le même type d'estimation réalisé avec des extraits de E.coli JM83 (pHTA2) portant un gène de δ-endotoxine de spécificité différente conduit à des valeurs 30 à 50 fois supérieures de la DL50 des extraits solubles, vis-à-vis de S.littoralis (135 à 350 μg/gramme de larve).

Les données qui précédent permettront aisément à l'homme de l'art d'élaborer des compositions larvicides actives avec les protéines de l'invention.

D'autres expériences de toxicité ont été réalisées en utilisant des larves au deuxième stade larvaire de M.brassicae, S.frugiperda et S.littoralis. Les résultats obtenus, exprimés en termes de LC 50 comme défini pour le tableau 1, sont donnés dans le tableau 2.

TABLEAU 2

ACTIVITE DES CLONES RECOMBINANTS
CONTRE LES LARVES D'INSECTES
DE LA FAMILLE DES NOCTUIDAE
M.BRASSICAE, S.FRUGIPERDA AND S.LITTORALIS

| SOUCHES ET PLASMIDES<br>LARVE D'INSECTE ET STADE | M. BRASSICAE<br>CL50 2ème STADE | S. FRUGIPERDA<br>CL50 2ème STADE | S. LITTORALIS<br>CL50 2ème STADE |
|---|---|---|---|
| JM 83 (pUC18) | NT | NT | NT |
| JM 83 (pHTA2) | > 1 | 0,51 | 0,9 |
| JM 83 (pHT671) | 0,02 | 0,5 | 0,03 |
| JM 83 (pHT71) | ND | ND | 0,03 |
| JM 83 (pHTA4) | > 1 | 0,54 | > 1 |

Il ressort de l'examen du tableau 2 que les extraits bactériens bruts du clone recombinant JM83 (pHT671) sont toxiques vis-à-vis de M.brassicae et de S.littoralis (les valeurs de CL50 sont respectivement de 0,02 et 0,03) et faiblement toxiques vis-à-vis de S.frugiperda (CL50 de 0,5).

Les extraits du clone recombinant E.coli JM83 (pHTA2) sont faiblement actifs à l'égard de S.frugiperda et de S.littoralis et pas du tout toxique à l'égard de M.brassicae. Les extraits du clone recombinant JM83

20

(pHTA4) ne sont pas toxiques vis-à-vis de M.brassicae et de S.littoralis et sont faiblement toxiques à l'égard de S.frugiperda.

Ces résultats confirment la forte toxicité spécifique des protéines obtenues à partir de pHT71 et de pH671 à l'égard de S.littoralis et montrent que cette classe de protéine de cristal est aussi très active à l'égard de M.brassicae.

EXEMPLE VI : Etude de la spécificité des polypeptides exprimés par les clones formés par introduction des plasmides pHT671 et pHT71 dans E.coli.

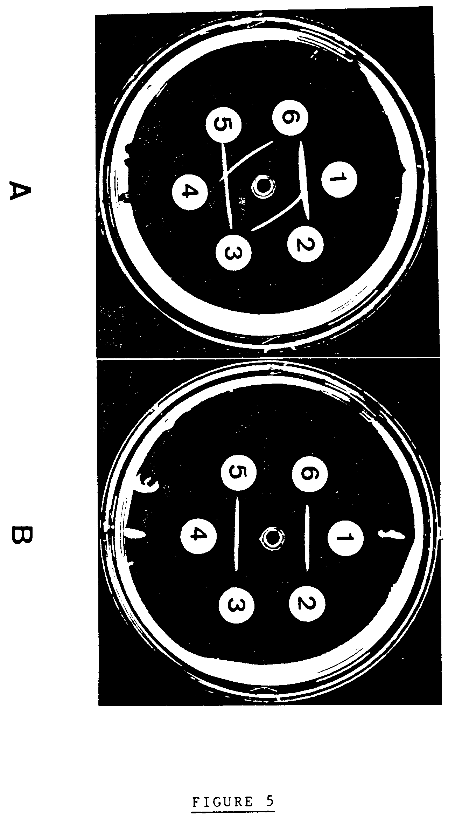

Cette étude a été réalisée grâce à des tests d'immuno-diffusion. Les résultats sont rapportés sur la figure 5 (qui comprend les figures 5A et 5B).

La mise en oeuvre de l'expérience d'immunodiffusion a été réalisée conformément au protocole suivant :

Des extraits solubles de protéines de clones E.coli contenant les plasmides pHT671, pHTA4, pHTA2 ou pHT71, pUC18 ont été placés respectivement dans les puits n° 2, 3, 4, 5, 6. Un échantillon d'un cristal purifié solubilisé de aizawai 7-29 a été placé dans le puits n° 1 afin de servir de contrôle positif.

Dans le test rapporté sur la figure 5A un antisérum contre toutes les δ-endotoxines de aizawai 7-29, contenant des anticorps de lapin dirigés contre les protéines du cristal solubilisées a été utilisé et placé dans le puits central.

Une ligne d'immunoprécipitation a été observée dans tous les cas excepté dans le cas de l'extrait de E.coli contenant le vecteur plasmidique seul (puits n° 6).

On a remarqué que les lignes d'immunoprécipitation issues des puits n° 4 et n° 5 croisent, ce qui montre que les produits codés par les plasmides pHTA2 et pHT71 respectivement présentent des déterminants anti-géniques différents.

Dans le test rapporté sur la figure 5B, l'anti-sérum utilisé contenait des anticorps polyclonaux de lapin contre les protéines du cristal de berliner 1715.

Une ligne d'immunoprécipitation a été observée avec les extraits de E.coli JM83 (pHTA4) (puits n° 3) JM83 (pHTA2) (puits n° 4). En revanche les clones E.coli JM83 (pHT71) (puits n° 5) JM83 (pHT671) (puits n° 2) ou JM83 (pUC9) (puits n° 6) ne donnent pas d'immunoprécipitation.

On peut en déduire que les gènes du cristal isolés dans pHTA4 et pHTA2 expriment des polypeptides ayant des déterminants antigéniques communs avec les protéines du cristal de berliner 1715, souche qui n'est pas spécifiquement active vis-à-vis de S.littoralis.

En revanche, les extraits bruts de E.coli contenant les plasmides pHT671 et pHT71 contiennent des polypeptides ayant des déterminants antigéniques communs avec les protéines du cristal de la souche aizawai 7.29, qui ne sont pas liés sur le plan immunogène avec les protéines du cristal de la souche berliner 1715.

Ces résultats confirment ceux donnés précédemment en rapport avec la spécificité des gènes isolés dans les plasmides pHT71 et pHT671.

Des essais de précipitation antigène-anticorps ont permis de déterminer le niveau d'expression des gènes de δ-endotoxine dans différents clones recombinants.

Les résultats obtenus ont montré que la protéine du cristal représente entre 7 et 10% de protéines cellulaires totales de E.coli, JM83 (pHTA2), entre 2 et 3% dans E.coli JM83 (pHT671) et entre 0,5 et 1% dans E.coli JM83 (pHTA4) et E.coli, JM83 (pHT71).

Les références bibliographiques dont il est question dans les exemples sont les suivantes :

(1)KLIER, A.F., LECADET, M-M. and DEDONDER, R., 1973, Sequential modifications of RNA polymerase during sporogenesis in Bacillus thuringiensis, Eur. J. Biochem., 36 : 317-327.
(2)MANIATIS, T., FRITSCH, E.F., SAMBROOK, J., 1982, Molecular cloning: A laboratory manual. Cold Spring Harbor Laboratory Press, New-York
(3)VIEIRA, J. and MESSING, J., 1982, The pUC plasmids, and M13mp7 derived system for insertion mutagenesis and sequencing with synthetic universal primers, Gene, 19 : 259-268.
(4)LEDERBERG, E.M. and COHEN, S.N., 1974, Transformation of Salmonella thyphimurium by plasmid deoxyribonucleic acid, J. Bacteriol., 119 : 1072-1074.
(5)GRUNSTEIN, M. and HOGNESS, D.S., 1975, Colony hybridization, a method for the isolation of cloned DNAs that contain a specific gene, Proc. Natl. Acad. Sci. U.S.A., 72 : 3961-3965.
(6)SOUTHERN, E.M., 1975, Detection of specific sequence among DNA fragments separated by gel electrophoresis, J. Molec. Biol., 98, 503-517.
(7)DENHARDT, D.T. 1976, A membrane filter taking for the detection of complementary DNA. Biochem. Biophys. Res. Comm., 23 : 641-646.

(8)SANGER, F., NICKLENS, S. and COULSON, A.R., 1977, DNA sequencing with chain terminating inhibitors. Proc. Natl. Acad. Sci. U.S.A., 74 : 5463-5467.

(9)DALE et al.(1985) A rapid single-stranded cloning strategy for producing a sequential series of overlapping clones for use in DNA, Plasmid 1985, 13 : 31-40

(10)LECADET.M.M. et MARTOURET D.1987, Host specificity of the Bacillus thuringiensis δ-endotoxin toward Lepidopteran species : Spodoptera littoralis Bdv and Pieris brassicae L, J. of Invert. Pathol., 49 - (n° 1) : 37-48.

(11)CHANG et al., 1979, High frequency transformation of Bacillus subtilis protoplasts by plasmid DNA- Mol Gen Genet 168:111 115

(12)HEIERSON et al., 1987, Transformation of vegetative cells of Bacillus thuringiensis by plasmid DNA, Journal of Bacteriology, Mar.1987, p.1147-1152,

(13)KLIER et al., 1983, Mating between Bacillus subtilis and Bacillus thuringiensis and transfer of cloned crystal genes, Mol Gen Genet (1983) 191:257 262

(14)LERECLUS et al., 1983, Isolation of a DNA, sequence related to several plasmids from Bacillus thuringiensis after a mating involving the Streptococcus faecalis plasmid pAMβ1, Mol Gen Genet (1983) 191:307-313

(15)UMBECK et al., 1987, Genetically transformed cotton (Gossypium hirsutum L.) plants - Biotechnology vol.5 March 1987.

(16)WONG et al., 1983, transcriptional and translational start sites for the Bacillus thuringiensis crystal protein gene. J. of Biol. Chem., 258 : 1960-1967.

(17)OBUKOWICZ M.et al (1986). Tn5 mediated integration of the δ-endotoxin gene from B. thuringiensis into the chromosome of root colonizing Pseudomonas. J. Bacteriol., 168, 982-989.

(18)SIMON, R. et al, (1983). A broad host range mobilization system for in vivo genetic engineering : transposon mutagenesis in Gram-negative bacteria. Biotechnology, 1, pp. 784-791.

(19)Schnepf et al,(1985) The amino acid sequence of a crystal protein from Bacillus thuringiensis deduced from the DNA base sequence. J BIOL Chem 260 : 6264-6372.

(20)Adang et al, (1985) characterized full-length and truncated plasmid clones of the crystal protein of Bacillus thuringiensis subsp. kurstaki HD-73 and their toxicity to Manduca sexta. Gene 36 : 289-300.

(21)Wabiko et al,(1986) Bacillus thuringiensis entomocidal protoxin gene sequence and gene product analysis. DNA 5 : 305-314.

(22)Hofte et al,(1986) Structural and functional analysis of a cloned δ-endotoxin gene of Bacillus thuringiensis berliner 1715. Eur J Biochem 161 : 273-280.

(23)Shibano et al,(1986) Complete structure of an insecticidal crystal protein gene from Bacillus thuringiensis. In : Bacillus molecular genetics and biotechnology applications. J.Ganesan, A.T., Hoch, J.A.(eds). Academic Press 307-320.

(24)Oeda et al,(1987) Nucleotide sequence of the insecticidal protein gene of Bacillus thuringiensis strain aizawai IPL7 and its high-level expression in Escherichia coli. Gene 53 : 113-119.

**Revendications**

1. Séquence d'ADN codant pour au moins une partie de la région N-terminale d'un polypeptide toxique de manière spécifique vis-à-vis de larves de lépidoptères de la famille des Noctuelles, caractérisée en ce qu'elle contient un fragment d'ADN d'environ 3 kb correspondant au fragment de restriction HindIII - PstI provenant de B.thuringiensis souche aizawai 7.29.

2. Séquence d'ADN selon la revendication 1, caractérisée en ce que le fragment de restriction HindIII - PstI est capable d'hybrider avec les sondes 1, 2, 3 de pHTA2 rapportées sur la figure 2.

3. Séquence d'ADN selon la revendication 1 ou 2 caractérisée en ce qu'elle comporte des sites dans l'ordre suivant : HindIII - HincII - BglII - KpnI - HindIII - PstI.

4. Séquence d'ADN selon la revendication 1, caractérisée en ce qu'elle code pour un polypeptide capable de former un complexe immunologique avec des anticorps dirigés contre des polypeptides à activité larvicide vis-à-vis de S.littoralis.

5. Séquence d'ADN selon la revendication 1, caractérisée en ce qu'elle code pour polypeptide toxique de S.littoralis.

22

6.  Séquence d'ADN codant pour un polypeptide toxique vis-à-vis de larves de lépidoptères de la famille des Noctuelles, caractérisée en ce qu'elle comprend un fragment d'ADN correspondant au fragment de restriction HindIII - HincII d'environ 1.1 kb provenant de B.thuringiensis souche entomocidus 6-01 fusionné avec un fragment de restriction HincII - PstI d'environ 1.9 kb provenant de B.thuringiensis souche aizawai 729.

7.  Séquence d'ADN selon la revendication 6, caractérisée en ce qu'elle code pour un polypeptide capable de former un complexe immunologique avec des anticorps dirigés contre des polypeptides à activité larvicide vis-à-vis de S.littoralis.

8.  Séquence de nucléotides caractérisée en ce qu'elle a la capacité d'hybrider avec une sonde formée à partir de la séquence (I) présentant l'enchaînement suivant:

```
      52
GTC TAC TTG ACA GGG GTA GGA ACA TAA TCC GTC AAT TTT AAA TAT GGG GCA TAT ATT GAT
     112
ATT TTA TAA AAT TTG TTA CGT TTT TTG TAT TTT TTC ATA ACA TGT GTC ATA TGT ATT AAA
     172
TCG TGG TAA TGA AAA ACA GTA TCA AAC TAT CAG AAC TTT GGT AGT TTA ATA AAA AAA CGC
     232
AGG TAT TTT ATG GAG GAA AAT AAT CAA AAT CAA TGC ATA CCT TAC AAT TGT TTA AGT AAT
     292
CCT GAA GAA GTA CTT TTG GAT GGA GAA CGG ATA TCA ACT GGT AAT TCA TCA ATT GAT ATT
     352
TCT CTG TCA CTT GTT CAG TTT CTG GTA TCT AAC TTT GTA CCA GGG GGA GGA TTT TTA GTT
     412
GGA TTA ATA GAT TTT GTA TGG GGA ATA GTT CGC CCT TCT CAA TGG GAT GCA TTT CTA GTA
     472
CAA ATT GAA CAA TTA ATT AAT GAA AGA ATA GCT GAA TTT GCT AGG AAT GCT GCT ATT GCT
     532
AAT TTA GAA GGA TTA GGA AAC AAT TTC AAT ATA TAT GTG GAA GCA TTT AAA GAA TGG GAA
     592
GAA GAT CCT AAT AAT CCA GAA ACC AGG ACC AGA GTA ATT GAT CGC TTT CGT ATA CTT GAT
     652
GGG CTA CTT GAA AGG GAC ATT CCT TCG TTT CGA ATT TCT GGA TTT GAA GTA CCC CTT TTA
     712
TCC GTT TAT GCT CAA GCG GCC AAT CTG CAT CTA GCT ATA TTA AGA GAT TCT GTA ATT TTT
     772
GGA GAA AGA TGG GGA TTG ACA ACG ATA AAT GTC AAT GAA AAC TAT AAT AGA CTA ATT AGC
     832
CAT ATT GAT GAA TAT GCT GAT GAC TGT CCA AAT ACG TAT AAT CGG GGA TTA AAT AAT TTA
     892
CCG AAA TCT ACG TAT CAA GAT TGG ATA ACA TAT AAT CGA TTA CGG AGA GAC TTA ACA TTG
     952
ACT GTA TTA GAT ATC GCC GCT TTC TTT CCA AAC TAT GAC
```

cu à partir de la séquence (III) présentant l'enchaînement suivant :

ATG CTT CAA TAG AAT CTC AAA TCT CGA TGA CTG CTT AGT CTT TTT AAT ACT GTC TAC TTG ACA GGG GTA GGA ACA TAA TCG GTC AAT TT1

AAA TAT GGG GCA TAT ATT GAT ATT TTA TAA AAT TTG TTA CGT TTT TTG TAT TTT TTC ATA AGA TGT GTC ATA TGT ATT AAA TCG TGG TAA

TGA AAA ACA GTA TCA AAC TAT CAG AAC TTT GGT AGT TTA ATA AAA AAA CGG AGG TAT TTT ATG GAG GAA AAT AAT CAA AAT CAA TGC ATA

CCT TAC AAT TGT TTA AGT AAT CCT GAA GAA GTA CTT TTG GAT GGA GAA CGG ATA TCA ACT GGT AAT TCA TCA ATT GAT ATT TCT CTG TCA

CTT GTT CAG TTT CTG GTA TCT AAC TTT GTA CCA GGG GGA GGA TTT TTA GTT GGA TTA ATA GAT TTT GTA TGG GGA ATA GTT GGC CCT TCT

CAA TGG GAT GCA TTT CTA GTA CAA ATT GAA CAA TTA ATT AAT GAA AGA ATA GCT GAA TTT GCT AGG AAT GCT GCT ATT GCT AAT TTA GAA

GGA TTA GGA AAC AAT TTC AAT ATA TAT GTG GAA GCA TTT AAA GAA TGG GAA GAA GAT CCT AAT AAT CCA GCA ACC AGG ACC AGA GTA ATT

GAT CGC TTT CGT ATA CTT GAT GGG CTA CTT GAA AGG GAC ATT CCT TCG TTT CGA ATT TCT GGA TTT GAA GTA CCC CTT TTA TCC GTT TAT

GCT CAA GCG GCC AAT CTG CAT CTA GCT ATA TTA AGA GAT TCT GTA ATT TTT GGA GAA AGA TGG GGA TTG ACA ACG ATA AAT GTC AAT GAA

AAC TAT AAT AGA CTA ATT AGG CAT ATT GAT GAA TAT GCT GAT CAC TGT GCA AAT ACG TAT AAT CGG GGA TTA AAT AAT TTA CCG AAA TCT

ACG TAT CAA GAT TGG ATA ACA TAT AAT CGA TTA CGG AGA GAC TTA ACA TTG ACT GTA TTA GAT ATC GCC GCT TTC TTT CCA AAC TAT GAC

991

AAT AGG AGA TAT CCA ATT CAG CCA GTT GGT CAA CTA ACA AGG GAA GTT TAT ACG GAC CCA TTA ATT AAT TTT AAT CCA CAG TTA CAG TCT

1081

GTA GCT CAA TTA CCT ACT TTT AAC GTT ATG GAG AGC AGC GCA ATT AGA AAT CCT CAT TTA TTT GAT ATA TTG AAT AAT CTT ACA ATC TTT

1171

ACG GAT TGG TTT AGT GTT GGA CGC AAT TTT TAT TGG GGA GGA CAT CGA GTA ATA TCT AGC CTT ATA GGA GGT GGT AAC ATA ACA TCT CCT

1261

ATA TAT GGA AGA GAG GCG AAC CAG GAG CCT CCA AGA TCC TTT ACT TTT AAT GGA CCG GTA TTT AGG ACT TTA TCA ATT CCT ACT TTA CGA

1351

TTA TTA CAG CAA CCT TGC CAG CGC CAC CAT TTT AAT TTA CGT GGT GGT GAA GGA GTA GAA TTT TCT ACA CCT ACA AAT AGC TTT ACG TAT

1441

CGA GGA AGA GGT ACG GTT GAT TCT TTA ACT GAA TTA CCG CCT GAG GAT AAT AGT GTG CCA CCT CGC GAA GGA TAT AGT CAT CGT TTA TGT

1531

CAT GCA ACT TTT GTT CAA AGA TCT GGA ACA CCT TTT TTA ACA ACT GGT GTA GTA TTT TCT TGG ACG CAT CGT AGT GCA ACT CTT ACA AAT

1621

ACA ATT GAT CCA GAG AGA ATT AAT CAA ATA CCT TTA GTG AAA GGA TTT AGA GTT TGG GGG GGC ACC TCT GTC ATT ACA GGA CCA GGA TTT

1711

ACA GGA GGG GAT ATC CTT CGA AGA AAT ACC TTT GGT GAT TTT GTA TCT CTA CAA GTC AAT ATT AAT TCA CCA ATT ACC CAA AGA TAC CGT

1801

TTA AGA TTT CGT TAC GCT TCC AGT AGG GAT GCA CGA GTT ATA GTA TTA ACA GGA GCG GCA TCC ACA GGA GTG GGA GGC CAA GTT AGT GTA

```
1861  GGT ATG CCT CTT CAG AAA ACT ATG GAA ATA GGG TGG AAC TTA ACA TCT AGA TAT ACC GAT TTT AGT AAT CCT TTT TCA TCT
2071  ACA CCT AAT CCA GAT ATA ATT GGG ATA AGT GAA CAA CCT CTA TTT GGT TCT ATT AGT AGC GGT GAA CTT TAT AGT AAT ATT
2161  GGA ATT ATT CTA GCA GAT GCA ACA TTT GAA GCA GAA TCT GAT TTA GAT GTG GAT GCC CTG TTT ACT TCT TCC AAT
2251  CAA ATC GCG TTA AAA ACC GAT GTG ACG GAT CAT CAT GAA GTA TCC AAT TTA GTG GAT TGT TTA TCA GAA TTT TGT CTG GAT
2341  GAA AAG TTA GAA TTG TCC GAG AAA GTC AAA CAT GCG AAG CCA CTC AGT ACC ATC CAA GAT TTA CTT CAA GGA GAT GAG GGG ATC
2431  AAT AGA CAA CCA CAC CGT GGC TGG AGA GGA AGT ACA GAT ATT ACC ATC CAA GGA GGA GAT GAG GTA TTC AAA GAT TAC GTC ACA CTA
2521  CCG GGT ACC GTT GAT GAG TGC TAT CCA ACG TAT TTA TAT CAG AAA ATA GAT GCT TAT ACC GGT TAT TTA AGA
2611  GCG TAT ATC GAA GAT AGT GAT TTA GAA ATC TAT TTG ATC GCG TAC AAT CGA TGC GCC CCA CAC CTT GAT CTA GAT
2701  TTA TGG CCG CTT TCA GCC CAA AGT CCA ATC GGA AAG TGT GGA GAA CCG AAT CCT GAT CTA GAT

      TGT TCC TGC AG
```

9. Séquence de nucléotides codant pour un polypeptide toxique de manière spécifique vis-à-vis des larves de lépidoptères de la famille des Noctuelles, de préférence vis-à-vis de S.littoralis, caractérisée en ce qu'elle comprend l'enchaînement (I) ou (III) défini à la revendication 8.

**10.** Séquence de nucléotides selon la revendication 8 ou 9, caractérisée en ce qu'elle présente un codon d'initiation ATG situé en position 241.

**11.** Séquence selon l'une quelconque des revendications 8 à 10 caractérisée par un site de liaison aux ribosomes GGAGG en positions 230 à 234.

**12.** Séquence selon l'une des revendications 9 à 11, caractérisée en ce qu'elle comporte la séquence comprise entre les nucléotides en position 137 et 177 (position -103 à -63 en amont du codon d'initiation ATG) qui est homologue à raison d'environ au moins 70% à la région présente en amont du gène du cristal de la souche kurstaki-HD1 Dipel (BTK) qui contient les trois promoteurs BtI, BtII et Ec fonctionnels dans B.thuringiensis et E. coli respectivement.

**13.** Séquence de nucléotides, caractérisée en ce qu'elle code pour un polypeptide comprenant la séquence (II) d'acides aminés ci-après:

```
MET GLU GLU ASN ASN GLN ASN GLN CYS ILE PRO TYR ASN CYS LEU SER ASN

PRO GLU GLU VAL LEU LEU ASP GLY GLU ARG ILE SER THR GLY ASN SER SER ILE ASP ILE

SER LEU SER LEU VAL GLN PHE LEU VAL SER ASN PHE VAL PRO GLY GLY GLY PHE LEU VAL

GLY LEU ILE ASP PHE VAL TRP GLY ILE VAL GLY PRO SER GLN TRP ASP ALA PHE LEU VAL

GLN ILE GLU GLN LEU ILE ASN GLU ARG ILE ALA GLU PHE ALA ARG ASN ALA ALA ILE ALA

ASN LEU GLU GLY LEU GLY ASN ASN PHE ASN ILE TYR VAL GLU ALA PHE LYS GLU TRP GLU

GLU ASP PRO ASN ASN PRO GLU THR ARG THR ARG VAL ILE ASP ARG PHE ARG ILE LEU ASP

GLY LEU LEU GLU ARG ASP ILE PRO SER PHE ARG ILE SER GLY PHE GLU VAL PRO LEU LEU

SER VAL TYR ALA GLN ALA ALA ASN LEU HIS LEU ALA ILE LEU ARG ASP SER VAL ILE PHE

GLY GLU ARG TRP GLY LEU THR THR ILE ASN VAL ASN GLU ASN TYR ASN ARG LEU ILE ARG

HIS ILE ASP GLU TYR ALA ASP HIS CYS ALA ASN THR TYR ASN ARG GLY LEU ASN ASN LEU

PRO LYS SER THR TYR GLN ASP TRP ILE THR TYR ASN ARG LEU ARG ARG ASP LEU THR LEU

THR VAL LEU ASP ILE ALA ALA PHE PHE PRO ASN TYR ASP
```

ou caractérisée en
ce qu'elle code pour un polypeptide comprenant la séquence (IV) d'acides aminés ci-après :

MET GLU GLU ASN ASN GLN ASN GLN CYS ILE

271
PRO TYR ASN CYS LEU SER ASN PRO GLU GLU VAL LEU LEU ASP GLY GLU ARG ILE SER THR GLY ASN SER SER ILE ASP ILE SER LEU SER

361
LEU VAL GLN PHE LEU VAL SER ASN PHE VAL PRO GLY GLY GLY PHE LEU VAL GLY LEU ILE ASP PHE VAL TRP GLY ILE VAL GLY PRO SER

451
GLN TRP ASP ALA PHE LEU VAL GLN ILE GLU GLN LEU ILE ASN GLU ARG ILE ALA GLU PHE ALA ARG ASN ALA ALA ILE ALA ASN LEU GLU

541
GLY LEU GLY ASN ASN PHE ASN ILE TYR VAL GLU ALA PHE LYS GLU TRP GLU GLU ASP PRO ASN ASN PRO ALA THR ARG THR ARG VAL ILE

631
ASP ARG PHE ARG ILE LEU ASP GLY LEU LEU GLU ARG ASP ILE PRO SER PHE ARG ILE SER GLY PHE GLU VAL PRO LEU LEU SER VAL TYR

721
ALA GLN ALA ALA ASN LEU HIS LEU ALA ILE LEU ARG ASP SER VAL ILE PHE GLY GLU ARG TRP GLY LEU THR THR ILE ASN VAL ASN GLU

811
ASN TYR ASN ARG LEU ILE ARG HIS ILE ASP GLU TYR ALA ASP HIS CYS ALA ASN THR TYR ASN ARG GLY LEU ASN ASN LEU PRO LYS SER

901
THR TYR GLN ASP TRP ILE THR TYR ASN ARG LEU ARG ARG ASP LEU THR LEU THR VAL LEU ASP ILE ALA ALA PHE PHE PRO ASN TYR ASP

EP 0 295 156 B1

991

ASN ARG ARG TYR PRO ILE GLN PRO VAL GLY GLN LEU THR ARG GLU VAL TYR THR ASP PRO LEU ILE ASN PHE ASN PRO GLN LEU GLN SER

1081
VAL ALA GLN LEU PRO THR PHE ASN VAL MET GLU SER SER ALA ILE ARG ASN PRO HIS LEU PHE ASP ILE LEU ASN ASN LEU THR ILE PHE

1171
THR ASP TRP PHE SER VAL GLY ARG ASN PHE TYR TRP GLY GLY HIS ARG VAL ILE SER SER LEU ILE GLY GLY GLY ASN ILE THR SER PRO

1261
ILE TYR GLY ARG GLU ALA ASN GLN GLU PRO PRO ARG SER PHE THR PHE ASN GLY PRO VAL PHE ARG THR LEU SER ILE PRO THR LEU ARG

1351
LEU LEU GLN GLN PRO CYS GLN ARG HIS HIS PHE ASN LEU ARG GLY GLY GLU GLY VAL GLU PHE SER THR PRO THR ASN SER PHE THR TYR

1441
ARG GLY ARG GLY THR VAL ASP SER LEU THR GLU LEU PRO PRO GLU ASP ASN SER VAL PRO PRO ARG GLU GLY TYR SER HIS ARG LEU CYS

1531
HIS ALA THR PHE VAL GLN ARG SER GLY THR PRO PHE LEU THR THR GLY VAL VAL PHE SER TRP THR HIS ARG SER ALA THR LEU THR ASN

1621
THR ILE ASP PRO GLU ARG ILE ASN GLN ILE PRO LEU VAL LYS GLY PHE ARG VAL TRP GLY GLY THR SER VAL ILE THR GLY PRO GLY PHE

1711
THR GLY GLY ASP ILE LEU ARG ARG ASN THR PHE GLY ASP PHE VAL SER LEU GLN VAL ASN ILE ASN SER PRO ILE THR GLN ARG TYR ARG

1801
LEU ARG PHE ARG TYR ALA SER SER ARG ASP ALA ARG VAL ILE VAL LEU THR GLY ALA ALA SER THR GLY VAL GLY GLY GLN VAL SER VAL

(151)
ASN MET PRO LEU GLN LYS THR MET GLU ILE GLY GLY,GLU ASN LEU THR SER ARG THR PHE ARG TYR ARG ASP PHE SER ASN PRO PHE SER PHE

(180)
ARG ALA ASN PRO ASP ILE ILE GLY ILE SER SER GLU GLN PRO LEU PHE GLY SER ILE SER SER GLU LEU TYR ILE ASP SER LYS ILE

(207)
GLU ILE ILE LEU ALA ASP ALA GLU PHE GLU SER ASP LEU GLU ARG ALA GLN ALA GLN ALA LEU VAL ASN ALA LEU PHE THR SER SER ASN

(216)
GLN ILE ILE GLY LEU LYS THR ASP VAL THR ASP TYR HIS ILE ASP SER GLN VAL SER ASN LEU VAL ASP CYS LEU SER ASP GLU PHE CYS LEU ASP

(225)
GLU LYS ARG GLU LEU SER GLU GLU LYS VAL LYS HIS ALA LYS ARG ASN LEU LEU GLN ASP PRO ASN PHE ARG GLY ILE

(224)
ASN ARG GLN PRO ASP ARG GLY TRP ARG GLY SER THR ASP ILE GLN ASP SER VAL PHE LYS GLU ASN TYR THR ARG TYR VAL THR LEU

(243)
PRO GLY THR VAL VAL ASP GLU CYS TYR PRO THR THR TYR GLN ILE ASP THR ILE LYS ILE VAL ALA TYR ARG ALA TYR TYR GLU LEU ARG

(252)
GLY TYR ILE GLU GLU ASP SER GLN ASP LEU GLU ILE TYR ASN ALA LYS HIS GLU ILE VAL ASN VAL PRO GLY THR GLY THR GLY

(261)
LEU TRP PRO LEU SER GLN ALA GLN ASP PRO ILE GLY LYS CYS GLY GLU PRO ASN ARG CYS ALA PRO HIS LEU GLU TRP ASN PRO ASP LEU ASP SER

(270)
CYS SER CYS

**15.** Plasmide selon la revendication 14 caractérisé en ce qu'il s'agit de pHT671 tel que représenté sur la figure 4, ou de pHT71 comprenant un fragment d'ADN HindIII - PstI constitué uniquement d'ADN provenant de la souche aizawai 7-29 cloné dans un vecteur pUC9 préalablement hydrolysé avec les enzymes HindIII et PstI.

**16.** Souches bactériennes modifiées, caractérisées en ce qu'après transformation elles comportent une séquence de nucléotides selon l'une des revendications 1 à 13.

**17.** Souche bactérienne selon la revendication 16, caractérisée en ce qu'elle comporte au moins un vecteur recombinant selon la revendication 14 ou 15.

**18.** Polypeptide toxique vis-à-vis des larves de lépidoptères et de manière préférentielle vis-à-vis de S.littoralis, caractérisé en ce qu'il est capable de former un complexe immunologique avec des anticorps dirigés contre des polypeptides à activité larvicide vis-à-vis de S.littoralis repondant à la séquence (II) ou à la séquence (IV) d'acides aminés.

**19.** Polypeptide selon la revendication 18, caractérisé en ce qu'il comprend la séquence (II) ou la séquence (IV) d'acides aminés définies dans la revendication 13.

**20.** Procédé d'obtention d'une séquence nucléotidique selon la revendication 1 codant pour au moins une partie de la région N-terminale d'un polypeptide toxique de manière spécifique vis-à-vis de larves de lépidoptères de la famille des Noctuelles, de préférence vis-à-vis de S.littoralis, caractérisé par les étapes suivantes :
- la réalisation d'une hybridation entre d'une part une séquence de nucléotides d'une souche de B.thuringiensis active contre S.littoralis et d'autre part, une ou plusieurs séquences de nucléotides utilisées comme sondes provenant de la partie 5' d'une part et de la partie 3' d'autre part, d'un fragment de restriction d'un gène d'une δ-endotoxine de B.thuringiensis souche aizawai 7-29, ces parties 5' et 3' codant respectivement pour la partie N-terminale et pour la partie COOH-terminale d'un polypeptide de 130 kd toxique vis-à-vis des lépidoptères et en particulier actif contre P.brassicae et inactif contre S.littoralis, ce gène ayant été cloné dans le plasmide recombinant pHTA2 tel que représenté sur la figure 2
- l'isolement du fragment,
- son clonage dans un vecteur, suivi de sa purification.

**21.** Procédé selon la revendication 20, caractérisé en ce que le fragment recombiné au vecteur dans l'étape de clonage est élaboré à partir d'au moins une séquence de nucléotides issue d'au moins un vecteur recombinant contenant une séquence de nucléotides d'au moins une souche de B.thuringiensis.

**22.** Procédé selon la revendication 21, caractérisé en ce que le fragment recombiné au vecteur dans l'étape de clonage est élaboré à partir de plusieurs séquences de nucléotides issues de vecteurs recombinants contenant des séquences de nucléotides d'au moins 2 souches différentes de B.thuringiensis, possédant les mêmes cartes de restriction et contenant elles-mêmes tout ou partie des séquences de nucléotides capables de coder pour un polypeptide actif de manière préférentielle vis-à-vis de S.littoralis.

**23.** Procédé selon la revendication 21, caractérisé en ce que le fragment recombiné au vecteur dans l'étape de clonage est élaboré à partir d'un fragment de restriction HindIII - PstI provenant de la souche aizawai 7-29.

**24.** Procédé selon la revendication 22, caractérisé en ce que le fragment recombiné au vecteur dans l'étape de clonage est élaboré à partir d'un fragment de restriction HindIII - HincII provenant de la souche entomocidus 6-01 et d'un fragment de restriction HincII - PstI provenant de la souche aizawai 7-29.

**25.** Procédé selon la revendication 20, caractérisé en ce que le fragment de restriction recombiné selon la revendication 23 est porté par un plasmide pHTA6 tel que représenté sur la figure 1 et les fragments de restriction recombinés selon la revendication 24, HindIII - HincII et HincII - PstI sont portés par les plasmides recombinants respectifs pHTE6 et pHTA6, lesdits plasmides pHTA6 et pHTE6 tel que représenté sur la figure 1 étant tels qu'isolés à l'aide d'une sonde constituée par un fragment PvuII de 2kb du plasmide pBT15-88 correspondant à la partie interne d'un gène du cristal chromosomique de la souche berliner 1715, à partir de clones transformants renfermant des séquences nucléotidiques issues de souches B.thuringiensis actives vis-à-vis de larves de lépidoptères inter-alia de S.littoralis.

26. Composition larvicide à activité préférentielle vis-à-vis de S.littoralis , caractérisée en ce qu'elle renferme une quantité efficace de polypeptide tel que défini dans l'une quelconque des revendications 18 et 19 exprimé par les séquences nucleotidiques selon l'une des revendications 1 à 13, le vecteur selon la revendication 14, ou le plasmide selon la revendication 15, ou la souche bactérienne selon l'une quelconque des revendications 16 ou 17.

27. Application des séquences de nucléotides selon l'une quelconque des revendications 1 à 13 pour produire un polypeptide toxique vis-à-vis de lépidoptères, de préférence S.littoralis, dans des microorganismes capables d'exprimer des vecteurs recombinants renfermant ces séquences tels que E.coli, B.subtilis, B.cereus ou B.thuringiensis.

28. Application selon la revendication 27, caractérisée en ce que les séquences de nucléotides sont introduites dans des microorganismes vivant dans l'environnement ou en association avec les plantes, tels que Pseudomonas, Azospirillum ou Rhizobium et capables d'exprimer des vecteurs recombinants renfermant ces séquences.

29. Application selon la revendication 27 ou 28, caractérisée en ce que les séquences de nucléotides sont introduites dans des microorganismes en association avec différents gènes de δ-endotoxine.

30. Application des séquences de nucléotides selon l'une quelconque des revendications 1 à 13 à la transformation des plantes sensibles à S.littoralis, caractérisée en ce qu'elle comprend le transfert et l'expression de ces séquences dans ces plantes.

31. Cellules végétales dont le génome, après transformation par un procédé non essentiellement biologique, possède de façon stable une séquence de nucléotides capable d'exprimer un polypeptide toxique vis-à-vis de S.littoralis, telle que définie dans l'une quelconque des revendications 1 à 13 et, cellules issues de leur division.

32. Plantes ayant en particulier pour prédateur S.littoralis, transformées par un procédé non essentiellement biologique, dont le génome possède de façon stable une séquence de nucléotides, telle que définie dans l'une quelconque des revendications 1 à 13, capable d'exprimer un polypeptide toxique vis-à-vis de S.littoralis et, plantes issues de leur reproduction, de leur multiplication, ou de croisements hybrides.

33. Plante ayant en particulier pour prédateur S.littoralis, possédant en plus de leurs caractères génotypiques et phénotypiques initiaux la propriété d'exprimer un polypeptide toxique répondant à la séquence d'acides aminés définie à la revendication 13, de manière préférentielle vis-à-vis S.littoralis, cette propriété résultant de l'insertion dans son génome par manipulation génétique d'une séquence de nucléotides capable d'exprimer ledit polypeptide.

34. Graine capable de donner une plante selon la revendication 32 ou 33 ou issue d'une telle plante, caractérisée en ce qu'elle a intégré dans son génome, par manipulation génétique une séquence de nucléotides selon l'une quelconque des revendications 1 à 13.

**Claims**

1. DNA sequence encoding at least a portion of the N-terminal region of a polypeptide specifically toxic against larvae of Lepidoptera of the Noctuidae family, characterized in that it contains a DNA fragment of about 3 kb corresponding to the HindIII - PstI restriction fragment derived from B. thuringiensis strain aizawai 7-29.

2. DNA sequence according to Claim 1, characterized in that the HindIII - PstI restriction fragment is capable of hybridizing with probes 1, 2, 3 of pHTA2 presented in Figure 2.

3. DNA sequence according to Claim 1 or 2, characterized in that it comprises sites in the following order: HindIII - HincII - BgIII - KpnI - HindIII - PstI.

4. DNA sequence according to Claim 1, characterized in that it encodes a polypeptide capable of forming an immunological complex with antibodies to polypeptides with larvicidal activity against S. littoralis.

5. DNA sequence according to Claim 1, characterized in that it encodes a toxic polypeptide of S. littoralis.

6. DNA sequence encoding a polypeptide which is toxic against larvae of Lepidoptera of the Noctuidae family, characterized in that it comprises a DNA fragment corresponding to the HindIII - HincII restriction fragment of about 1.1 kb derived from B. thuringiensis strain entomocidus 6-01 fused with a HincII - PstI restriction fragment of about 1.9 kb derived from B. thuringiensis strain aizawai 729.

7. DNA sequence according to Claim 6, characterized in that it encodes a polypeptide capable of forming an immunological complex with antibodies to polypeptides with larvicidal activity against S. littoralis.

8. Nucleotide sequence characterized in that it is capable of hybridizing with a probe formed from the sequence (I) exhibiting the following configuration:

```
        52
GTC TAC TTG ACA GGG GTA GGA ACA TAA TGG GTC AAT TTT AAA TAT GGG GCA TAT ATT GAT
        112
ATT TTA TAA AAT TTG TTA CGT TTT TTG TAT TTT TTC ATA AGA TGT GTC ATA TGT ATT AAA
        172
TGG TGG TAA TGA AAA ACA GTA TCA AAC TAT CAC AAC TTT GGT AGT TTA ATA AAA AAA CGG
        232
AGG TAT TTT ATG GAG GAA AAT AAT GAA AAT GAA TGG ATA GCT TAC AAT TGT TTA AGT AAT
        292
CCT GAA GAA GTA CTT TTG GAT GGA GAA CGG ATA TCA ACT GGT AAT TCA TCA ATT GAT ATT
        352
TCT CTG TCA CTT GTT CAG TTT CTG GTA TCT AAC TTT GTA CCA GGG GGA GGA TTT TTA GTT
        412
GGA TTA ATA GAT TTT GTA TGG GGA ATA GTT GGC CCT TCT CAA TGG GAT GCA TTT CTA GTA
        472
CAA ATT GAA CAA TTA ATT AAT GAA AGA ATA GCT GAA TTT GCT AGG AAT GCT GCT ATT GCT
        532
AAT TTA GAA GGA TTA GGA AAC AAT TTC AAT ATA TAT GTC GAA GCA TTT AAA GAA TGG GAA
        592
GAA GAT CCT AAT AAT CCA GAA ACC AGG ACC AGA GTA ATT GAT CGC TTT CGT ATA CTT GAT
        652
GGG CTA CTT GAA AGG GAC ATT CCT TCG TTT CGA ATT TCT GGA TTT GAA GTA CCC CTT TTA
        712
TCC GTT TAT GCT CAA GCG GCC AAT CTG CAT CTA GCT ATA TTA AGA GAT TCT GTA ATT TTT
        772
GGA GAA AGA TGG GGA TTG ACA ACG ATA AAT GTC AAT GAA AAC TAT AAT AGA CTA ATT AGG
        832
CAT ATT GAT GAA TAT GCT GAT CAC TGT GCA AAT ACG TAT AAT CGG GGA TTA AAT AAT TTA
        892
CCG AAA TCT ACG TAT CAA GAT TGG ATA ACA TAT AAT CGA TTA CGG AGA GAC TTA ACA TTG
        952
ACT GTA TTA GAT ATC GCC GCT TTC TTT CCA AAC TAT GAC
```

or from the sequence (III) exhibiting the following configuration:

EP 0 295 156 B1

1 GAG CTT CAA TAG AAT CTC AAA TCT CGA TGA CTG CTT AGT CTT TTT AAT ACT GTC TAC TTG ACA GGG GTA GGA ACA TAA TCG GTC AAT TTT

91 AAA TAT GGG GCA TAT ATT GAT ATT TTA TAA AAT TTG TTA CGT TTT TTG TAT TTT TTC ATA AGA TGT GTC ATA TGT ATT AAA TCG TGG TAA

181 TGA AAA ACA GTA TCA AAC TAT CAG AAC TTT GGT AGT TTA ATA AAA AAA CGG AGG TAT TTT ATG GAG GAA AAT AAT CAA AAT CAA TGC ATA

271 CCT TAC AAT TGT TTA AGT AAT CCT GAA GAA GTA CTT TTG GAT GGA GAA CGG ATA TCA ACT GGT AAT TCA TCA ATT GAT ATT TCT CTG TCA

361 CTT GTT CAG TTT CTG GTA TCT AAC TTT GTA CCA GGG GGA GGA TTT TTA GTT GGA TTA ATA GAT TTT GTA TGG GGA ATA GTT GGC CCT TCT

451 CAA TGG GAT GCA TTT CTA GTA CAA ATT GAA CAA TTA ATT AAT GAA AGA ATA GCT GAA TTT GCT AGG AAT GCT GCT ATT GCT AAT TTA GAA

541 GGA TTA GGA AAC AAT TTC AAT ATA TAT GTG GAA GCA TTT AAA GAA TGG GAA GAA GAT CCT AAT AAT CCA GCA ACC AGG ACC AGA GTA ATT

631 GAT CGC TTT CGT ATA CTT GAT GGG CTA CTT GAA AGG GAC ATT CCT TCG TTT CGA ATT TCT GGA TTT GAA GTA CCC CTT TTA TCC GTT TAT

721 GCT CAA GCG GCC AAT CTG CAT CTA GCT ATA TTA AGA GAT TCT GTA ATT TTT GGA GAA AGA TGG GGA TTG ACA ACG ATA AAT GTC AAT GAA

811 AAC TAT AAT AGA CTA ATT AGG CAT ATT GAT GAA TAT GCT GAT CAC TGT GCA AAT ACG TAT AAT CGG GGA TTA AAT AAT TTA CCG AAA TCT

901 ACA TAT CAA GAT TGG ATA ACA TAT AAT CGA TTA CGG AGA GAC TTA ACA TTG ACT GTA TTA GAT ATC GCC GCT TTC TTT CCA AAC TAT GAC

EP 0 295 156 B1

991
AAT AGG AGA TAT CCA ATT CAG CCA GTT GGT CAA CTA ACA AGG GAA GTT TAT ACG GAC CCA TTA ATT AAT TTT AAT CCA CAG TTA CAG TCT

1081
GTA GCT CAA TTA CCT ACT TTT AAC GTT ATG GAG AGC AGC GCA ATT AGA AAT CCT CAT TTA TTT GAT ATA TTG AAT AAT CTT ACA ATC TTT

1171
ACG GAT TGG TTT AGT GTT GGA CGC AAT TTT TAT TGG GGA GGA CAT CGA GTA ATA TCT AGC CTT ATA GGA GGT GGT AAC ATA ACA TCT CCT

1261
ATA TAT GGA AGA GAG GCG AAC CAG GAG CCT CCA AGA TCC TTT ACT TTT AAT GGA CCG GTA TTT AGG ACT TTA TCA ATT CCT ACT TTA CGA

1351
TTA TTA CAG CAA CCT TGC CAG CGC CAC CAT TTT AAT TTA CGT GGT GGT GAA GGA GTA GAA TTT TCT ACA CCT ACA AAT AGC TTT ACG TAT

1441
CGA GGA AGA GGT ACG GTT GAT TCT TTA ACT GAA TTA CCG CCT GAG GAT AAT AGT GTG CCA CCT CGC GAA GGA TAT AGT CAT CGT TTA TGT

1531
CAT GCA ACT TTT GTT CAA AGA TCT GGA ACA CCT TTT TTA ACA ACT GGT GTA GTA TTT TCT TGG ACG CAT CGT AGT GCA ACT CTT ACA AAT

1621
ACA ATT GAT CCA GAG AGA ATT AAT CAA ATA CCT TTA GTG AAA GGA TTT AGA GTT TGG GGG GGC ACC TCT GTC ATT ACA GGA CCA GGA TTT

1711
ACA GGA GGG GAT ATC CTT CGA AGA AAT ACC TTT GGT GAT TTT GTA TCT CTA CAA GTC AAT ATT AAT TCA CCA ATT ACC CAA AGA TAC CGT

1801
TTA AGA TTT CGT TAC GCT TCC AGT AGG GAT GCA CGA GTT ATA GTA TTA ACA GGA GCG GCA TCC ACA GGA GTG GGA GGC CAA GTT AGT GTA

9. Nucleotide sequence encoding a polypeptide specifically toxic against larvae of Lepidoptera of the Noctuidae family, preferentially against S. littoralis, characterized in that it comprises the configuration (I) or (III) defined in Claim 8.

10. Nucleotide sequence according to Claim 8 or 9, characterized in that it has an ATG initiation codon situated at position 241.

11. Sequence according to any one of Claims 8 to 10, characterized by a ribosome binding site GGAGG at positions 230 to 234.

12. Sequence according to one of Claims 9 to 11, characterized in that it comprises the sequence between the nucleotides at position 137 and 177 (position -103 to -63 upstream of the ATG initiation codon) which is homologous in an amount of about at least 70 % to the region present upstream of the crystal gene of the kurstaki HD1 Dipel (BTK) strain which contains the three promoters BtI, BtII and Ec which are functional in B. thuringiensis and E. coli respectively.

13. Nucleotide sequence characterized in that it encodes a polypeptide comprising the following amino acid sequence (II):

MET GLU GLU ASN ASN GLN ASN GLN CYS ILE PRO TYR ASN CYS LEU SER ASN

PRO GLU GLU VAL LEU LEU ASP GLY GLU ARG ILE SER THR GLY ASN SER SER ILE ASP ILE

SER LEU SER LEU VAL GLN PHE LEU VAL SER ASN PHE VAL PRO GLY GLY GLY PHE LEU VAL

GLY LEU ILE ASP PHE VAL TRP GLY ILE VAL GLY PRO SER GLN TRP ASP ALA PHE LEU VAL

GLN ILE GLU GLN LEU ILE ASN GLU ARG ILE ALA GLU PHE ALA ARG ASN ALA ALA ILE ALA

ASN LEU GLU GLY LEU GLY ASN ASN PHE ASN ILE TYR VAL GLU ALA PHE LYS GLU TRP GLU

GLU ASP PRO ASN ASN PRO GLU THR ARG THR ARG VAL ILE ASP ARG PHE ARG ILE LEU ASP

GLY LEU LEU GLU ARG ASP ILE PRO SER PHE ARG ILE SER GLY PHE GLU VAL PRO LEU LEU

SER VAL TYR ALA GLN ALA ALA ASN LEU HIS LEU ALA ILE LEU ARG ASP SER VAL ILE PHE

GLY GLU ARG TRP GLY LEU THR THR ILE ASN VAL ASN GLU ASN TYR ASN ARG LEU ILE ARG

HIS ILE ASP GLU TYR ALA ASP HIS CYS ALA ASN THR TYR ASN ARG GLY LEU ASN ASN LEU

PRO LYS SER THR TYR GLN ASP TRP ILE THR TYR ASN ARG LEU ARG ARG ASP LEU THR LEU

THR VAL LEU ASP ILE ALA ALA PHE PHE PRO ASN TYR ASP

or characterized in that it encodes a polypeptide comprising the following sequence (IV) of amino acids:

EP 0 295 156 B1

MET GLU GLU ASN ASN GLN ASN GLN CYS ILE

271
PRO TYR ASN CYS LEU SER ASN PRO GLU GLU VAL LEU LEU ASP GLY GLU ARG ILE SER THR GLY ASN SER SER ILE ASP ILE SER LEU SER

361
LEU VAL GLN PHE LEU VAL SER ASN PHE VAL PRO GLY GLY GLY PHE LEU VAL GLY LEU ILE ASP PHE VAL TRP GLY ILE VAL GLY PRO SER

451
GLN TRP ASP ALA PHE LEU VAL GLN ILE GLU GLN LEU ILE ASN GLU ARG ILE ALA GLU PHE ALA ARG ASN ALA ALA ILE ALA ASN LEU GLU

541
GLY LEU GLY ASN ASN PHE ASN ILE TYR VAL GLU ALA PHE LYS GLU TRP GLU GLU ASP PRO ASN ASN PRO ALA THR ARG THR ARG VAL ILE

631
ASP ARG PHE ARG ILE LEU ASP GLY LEU LEU GLU ARG ASP ILE PRO SER PHE ARG ILE SER GLY PHE GLU VAL PRO LEU LEU SER VAL TYR

721
ALA GLN ALA ALA ASN LEU HIS LEU ALA ILE LEU ARG ASP SER VAL ILE PHE GLY GLU ARG TRP GLY LEU THR THR ILE ASN VAL ASN GLU

811
GSA TYR ASN ARG LEU ILE ARG HIS ILE ASP GLU TYR ALA ASP HIS CYS ALA ASN THR TYR ASN ARG GLY LEU ASN ASN LEU PRO LYS SER

901
THR TYR GLN ASP TRP ILE THR TYR ASN ARG LEU ARG ARG ASP LEU THR LEU THR VAL LEU ASP ILE ALA ALA PHE PHE PRO ASN TYR ASP

991

ASN ARG ARG TYR PRO ILE GLN PRO VAL GLY GLN LEU THR ARG GLU VAL TYR THR ASP PRO LEU ILE ASN PHE ASN PRO GLN LEU GLN SER

1081
VAL ALA GLN LEU PRO THR PHE ASN VAL MET GLU SER SER ALA ILE ARG ASN PRO HIS LEU PHE ASP ILE LEU ASN ASN LEU THR ILE PHE

1171
THR ASP TRP PHE SER VAL GLY ARG ASN PHE TYR TRP GLY GLY HIS ARG VAL ILE SER SER LEU ILE GLY GLY GLY ASN ILE THR SER PRO

1261
ILE TYR GLY ARG GLU ALA ASN GLN GLU PRO PRO ARG SER PHE THR PHE ASN GLY PRO VAL PHE ARG THR LEU SER ILE PRO THR LEU ARG

1351
LEU LEU GLN GLN PRO CYS GLN ARG HIS HIS PHE ASN LEU ARG GLY GLY GLU GLY VAL GLU PHE SER THR PRO THR ASN SER PHE THR TYR

1441
ARG GLY ARG GLY THR VAL ASP SER LEU THR GLU LEU PRO PRO GLU ASP ASN SER VAL PRO PRO ARG GLU GLY TYR SER HIS ARG LEU CYS

1531
HIS ALA THR PHE VAL GLN ARG SER GLY THR PRO PHE LEU THR THR GLY VAL VAL PHE SER TRP THR HIS ARG SER ALA THR LEU THR ASN

1621
THR ILE ASP PRO GLU ARG ILE ASN GLN ILE PRO LEU VAL LYS GLY PHE ARG VAL TRP GLY GLY THR SER VAL ILE THR GLY PRO GLY PHE

1711
THR GLY GLY ASP ILE LEU ARG ARG ASN THR PHE GLY ASP PHE VAL SER LEU GLN VAL ASN ILE ASN SER PRO ILE THR GLN ARG TYR ARG

1801
LEU ARG PHE ARG TYR ALA SER SER ARG ASP ALA ARG VAL ILE VAL LEU THR GLY ALA ALA SER THR GLY VAL GLY GLY GLN VAL SER VAL

```
1191  GLN MET PRO LEU GLN GLN LYS THR MET GLU ILE GLY.GLU ASN LEU THR SER ARG TYR THR THR ASP PHE ARG TYR SER.ASN PRO PHE SER PHE
1301  ARG ALA ASN PRO ASP ILE ILE GLY ILE SER GLU GLN GLN PRO LEU PHE GLU GLY ILE SER SER ILE SER SER GLY LEU TYR ILE ASP LYS CYS ILE
2071  GLU ILE ILE LEU ALA ASP THR PHE GLU ALA.GLU SER ASP LEU GLU SER GLY ASP LEU GLU ALA GLN LYS ALA GLN LEU PHE ILE SER SER ASN
2161  GLN ILE GLY LYS LEU LEU THR ASP VAL THR ASP TYR.HIS ILE ASP TYR.HIS ILE ASP GLN VAL SER ASN LEU VAL ASP GLU GLU CYS LEU ASP
2251  GLU LYS ARG GLU LEU SER LEU LYS VAL LYS HIS ALA LYS ARG LEU ARG GLU ASN.GLU LEU LEU GLN ARG ASP PRO GLU PHE CYS LEU ASP
2341  ASN ARG GLN PRO ASP ARG GLY TRP ARG GLY SER THR ASP ILE THR ILE GLN GLY ALA SER ASP ASP VAL PHE LYS GLU TYR VAL PRO ASN PHE ARG GLY ILE
2431  PRO GLY TYR THR VAL ASP GLU CYS TYR TYR PRO THR TYR LEU TYR PRO GLU SER LYS LEU LYS ILE LYS ALA TYR THR ARG GLU VAL THR LEU
2521  GLY THR TYR ILE GLU ASP SER GLN ALA ASP LEU GLU ILE TYR LEU ILE ALA LYS ASN ALA LYS HIS GLU ILE HIS GLU ILE VAL ASN VAL PRO GLY GLU LEU LEU ARG
2611  LEU TRP PRO LEU SER ALA GLN SER PRO ILE GLY LYS CYS GLY LYS PRO ILE GLY LYS CYS GLY PRO ALA PRO HIS LEU GLU TRP ASN VAL PRO GLY THR GLY SER
2701  CYS SER CYS
```

**14.** Recombinant expression and cloning vector containing at least part of the nucleotide sequence as defined in any one of Claims 1 to 13.

**15.** Plasmid according to Claim 14, characterized in that it is pHT671 as represented in Figure 4, or pHT71 comprising a HindIII - PstI DNA fragment consisting solely of DNA derived from the aizawai 7-29 strain, cloned into a vector pUC9 previously hydrolysed with the enzymes HindIII and PstI.

**16.** Modified bacterial strains, characterized in that after transformation, they contain a nucleotide sequence according to one of Claims 1 to 13.

**17.** Bacterial strain according to Claim 16, characterized in that it contains at least one recombinant vector according to Claim 14 or 15.

**18.** Polypeptide toxic against the larvae of Lepidoptera and preferentially against S. littoralis, characterized in that it is capable of forming an immunological complex with antibodies to polypeptides with larvicidal activity against S. littoralis corresponding to the amino acid sequence (II) or (IV).

**19.** Polypeptide according to Claim 18, characterized in that it comprises the amino acid sequence (II) or (IV) defined in Claim 13.

**20.** Process for the production of a nucleotide sequence according to Claim 1 encoding at least part of the N-terminal region of a polypeptide specifically toxic against larvae of Lepidoptera of the Noctuidae family, preferentially against S. littoralis, characterized by the following steps:
- performing a hybridization between, on the one hand, a nucleotide sequence from a B. thuringiensis strain active against S. littoralis and, on the other hand, one or more nucleotide sequences, used as probes, derived, on the one hand, from the 5' part and, on the other hand, from the 3' part of a restriction fragment of a B. thuringiensis strain aizawai 7-29 $\delta$-endotoxin gene, these 5' and 3' parts respectively encoding the $NH_2$-terminal part and the COOH-terminal part of a 130 kd polypeptide toxic against Lepidoptera, and in particular active against P. brassicae and inactive against S. littoralis, this gene having been cloned into the recombinant plasmid pHTA2, as represented in Figure 2,
- isolating the fragment,
- its cloning into a vector, followed by its purification.

**21.** Process according to Claim 20, characterized in that the fragment recombined with the vector in the cloning step is prepared from at least one nucleotide sequence obtained from at least one recombinant vector containing a nucleotide sequence from at least one strain of B. thuringiensis.

**22.** Process according to Claim 21, characterized in that the fragment recombined with the vector in the cloning step is prepared from several nucleotide sequences obtained from recombinant vectors containing nucleotide sequences from at least 2 different strains of B. thuringiensis, possessing the same restriction maps and containing themselves all or part of the nucleotide sequences capable of encoding a polypeptide active preferentially against S. littoralis.

**23.** Process according to Claim 21, characterized in that the fragment recombined with the vector in the cloning step is prepared from a HindIII - PstI restriction fragment derived from the aizawai 7-29 strain.

**24.** Process according to Claim 22, characterized in that the fragment recombined with the vector in the cloning step is prepared from a HindIII - HincII restriction fragment derived from the entomocidus 6-01 strain and from a HincII - PstI restriction fragment derived from the aizawai 7-29 strain.

**25.** Process according to Claim 20, characterized in that the recombined restriction fragment according to Claim 23 is carried by a plasmid pHTA6 as represented in Figure 1 and the recombined restriction fragments according to Claim 24, HindIII - HincII and HincII - PstI, are carried by the respective recombinant plasmids pHTE6 and pHTA6, the said plasmids pHTA6 and pHTE6 as represented in Figure 1 being as isolated with the aid of a probe consisting of a 2kb PvuII fragment of the plasmid pBT15-88 corresponding to the inner portion of a chromosomal crystal gene from the berliner 1715 strain, from transformant clones containing nucleotide sequences obtained from strains of B. thuringiensis active against larvae of Lepidoptera, inter alia of S. littoralis.

**26.** Larvicidal composition active preferentially against S. littoralis, characterized in that it contains an effective quantity of polypeptide as defined in one of Claims 18 and 19 expressed by the nucleotide sequences according to one of Claims 1 to 13, the vector according to Claim 14, or the plasmid according to Claim 15 or the bacterial strain according to either one of Claims 16 and 17.

**27.** Application of the nucleotide sequences according to any one of Claims 1 to 13 for producing a polypeptide toxic against Lepidoptera, preferably S. littoralis, in microorganisms capable of expressing recombinant vectors containing these sequences such as E. coli, B. subtilis, B. cereus or B. thuringiensis.

**28.** Application according to Claim 27, characterized in that the nucleotide sequences are introduced into microorganisms living in the environment or in association with plants, such as Pseudomonas, Azospirillum or Rhizobium and capable of expressing recombinant vectors containing these sequences.

**29.** Application according to Claim 27 or 28, characterized in that the nucleotide sequences are introduced into microrganisms in association with different δ-endotoxin genes.

**30.** Application of the nucleotide sequences according to any one of Claims 1 to 13 to the transformation of S. littoralis-sensitive plants, characterized in that it comprises the transfer and expression of these sequences in these plants.

**31.** Plant cells whose genome, after transformation by a non-essential biological process, stably possesses a nucleotide sequence capable of expressing a polypeptide toxic against S. littoralis, as defined in any one of Claims 1 to 13, and cells obtained from their division.

**32.** Plants having in particular as predator S. littoralis, which are transformed by a non-essentially biological process, whose genome stably possesses a nucleotide sequence as defined in any one of Claims 1 to 13, capable of expressing a polypeptide toxic against S. littoralis and, plants obtained from their reproduction, from their multiplication or from hybrid crossings.

**33.** Plant having in particular as predator S. littoralis, possessing in addition to their original genotypic and phenotypic characters the property of expressing a polypeptide corresponding to the amino acid sequence defined in Claim 13, toxic preferentially against S. littoralis, this property resulting from the insertion into its genome, by genetic engineering, of a nucleotide sequence capable of expressing the said polypeptide.

**34.** Seed capable of yielding a plant according to Claim 32 or 33 or obtained from such a plant, characterized in that it has, integrated into its genome, by genetic engineering, a nucleotide sequence according to any one of Claims 1 to 13.

**Patentansprüche**

**1.** DNA-Sequenz, die für mindestens einen Teil der N-terminalen Region eines Polypeptids kodiert, das in spezifischer Weise gegenüber Larven von Schmetterlingen der Familie der Nachtfalter toxisch ist, dadurch **gekennzeichnet, daß**
sie ein DNA-Fragment von ca. 3 kb enthält, das dem Restriktionsfragment HindIII-PstI entspricht, welches aus B. thuringiensis, Stamm aizawai 7-29, stammt.

**2.** DNA-Sequenz gemäß Anspruch 1,
dadurch **gekennzeichnet, daß**
das Restriktionsfragment HindIII-PstI dazu befähigt ist, mit den Sonden 1, 2 und 3 von pHTA2 zu hybridisieren, welche auf Fig. 2 dargestellt sind.

**3.** DNA-Sequenz gemäß Anspruch 1 oder 2,
dadurch **gekennzeichnet, daß**
sie Stellen in der folgenden Reihenfolge enthält:
HindIII - HincII - BglII - KpnI - HindIII - PstI.

**4.** DNA-Sequenz gemäß Anspruch 1,
dadurch **gekennzeichnet,** daß
sie für ein Polypeptid kodiert, das dazu befähigt ist, einen immunologischen Komplex mit Antikörpern zu bilden, die gegen Polypeptide mit larvizider Wirksamkeit gegenüber S.littoralis gerichtet sind.

**5.** DNA-Sequenz gemäß Ansrpuch 1,
dadurch **gekennzeichnet,** daß
sie für ein von S. littoralis toxisches Polypeptid kodiert.

**6.** DNA-Sequenz, die für ein Polypeptid kodiert, das gegenüber Larven von Schmetterlingen der Familie der Nachtfalter toxisch ist,
dadurch **gekennzeichnet,** daß
sie ein DNA-Fragment enthält, das dem Restriktionsfragment HindIII - HincII von ca. 1,1 kb entspricht, welches aus B. thuringiensis, Stamm entomocidus 601, stammt, welcher mit einem Restriktionsfragment HincII-PstI von ca. 1,9 kb fusioniert ist, das wiederum aus B. thuringiensis, Stamm aizawai 7-29, stammt.

**7.** DNA-Sequenz gemäß Anspruch 6,
dadurch **gekennzeichnet,** daß
sie für ein Polypeptid kodiert, das dazu befähigt ist, einen immunologischen Komplex mit Antikörpern zu bilden, die gegen Polypeptide mit larvizider Wirksamkeit gegenüber S. littoralis gerichtet sind.

**8.** Sequenz von Nukleotiden,
dadurch **gekennzeichnet,** daß
sie die Befähigung besitzt, mit einer Sonde zu hybridisieren, die aus der Sequenz (I) gebildet ist, welche die folgende Kette aufweist:

```
                52
GTC TAC TTG ACA GGG GTA GGA ACA TAA TCG GTC AAT TTT AAA TAT GGG GCA TAT ATT GAT
                112
ATT TTA TAA AAT TTG TTA CGT TTT TTG TAT TTT TTC ATA AGA TGT GTC ATA TGT ATT AAA
                172
TCG TGG TAA TGA AAA ACA GTA TCA AAC TAT CAG AAC TTT GGT AGT TTA ATA AAA AAA CGC
                232
AGG TAT TTT ATG GAG GAA AAT AAT CAA AAT CAA TGC ATA CCT TAC AAT TGT TTA AGT AAT
                292
CCT GAA GAA GTA CTT TTG GAT GGA GAA CGG ATA TCA ACT GGT AAT TCA TCA ATT GAT ATT
                352
TCT CTG TCA CTT GTT CAG TTT CTG GTA TCT AAC TTT GTA CCA GGG GGA GGA TTT TTA GTT
                412
GGA TTA ATA GAT TTT GTA TGG GGA ATA GTT GGC CCT TCT CAA TGG GAT GCA TTT CTA GTA
                472
CAA ATT GAA CAA TTA ATT AAT GAA AGA ATA GCT GAA TTT GCT AGG AAT GCT GCT ATT GCT
                532
AAT TTA GAA GGA TTA GGA AAC AAT TTC AAT ATA TAT GTG GAA GCA TTT AAA GAA TGG GAA
                592
GAA GAT CCT AAT AAT CCA GAA ACC AGG ACC AGA GTA ATT GAT CGC TTT CGT ATA CTT GAT
                652
GGG CTA CTT GAA AGG GAC ATT CCT TCG TTT CGA ATT TCT GGA TTT GAA GTA CCC CTT TTA
                712
TCC GTT TAT GCT CAA GCG GCC AAT CTG CAT CTA GCT ATA TTA AGA GAT TCT GTA ATT TTT
                772
GGA GAA AGA TGG GGA TTG ACA ACG ATA AAT GTC AAT GAA AAC TAT AAT AGA CTA ATT AGC
                832
CAT ATT GAT GAA TAT GCT GAT GAC TGT GCA AAT ACG TAT AAT CGG GGA TTA AAT AAT TTA
                892
CCG AAA TCT ACG TAT CAA GAT TGG ATA ACA TAT AAT CGA TTA CGG AGA GAC TTA ACA TTG
                952
ACT GTA TTA GAT ATC GCC GCT TTC TTT CCA AAC TAT GAC
```

oder welche Sonde aus der Sequenz (III) gebildet ist, die die folgende Kette aufweist:

1
AAG CTT CAA TAG AAT CTC AAA TCT CGA TGA CTG CTT AGT CTT TTT AAT ACT GTC TAC TTG ACA GGG GTA GGA ACA TAA TCG GTC AAT TTT

91
AAA TAT GGG GCA TAT ATT GAT ATT TTA TAA AAT TTG TTA CGT TTT TTG TAT TTT TTC ATA AGA TGT GTC ATA TGT ATT AAA TCG TGG TAA

181
TGA AAA ACA GTA TCA AAC TAT CAG AAC TTT GGT AGT TTA ATA AAA AAA CGG AGG TAT TTT ATG GAG GAA AAT AAT CAA AAT CAA TGC ATA

271
CCT TAC AAT TGT TTA AGT AAT CCT GAA GAA GTA CTT TTG GAT GGA GAA CGG ATA TCA ACT GGT AAT TCA TCA ATT GAT ATT TCT CTG TCA

361
CTT GTT CAG TTT CTG GTA TCT AAC TTT GTA CCA GGG GGA GGA TTT TTA GTT GGA TTA ATA GAT TTT GTA TGG GGA ATA GTT GGC CCT TCT

451
CAA TGG GAT GCA TTT CTA GTA CAA ATT GAA CAA TTA ATT AAT GAA AGA ATA GCT GAA TTT GCT AGG AAT GCT GCT ATT GCT AAT TTA GAA

541
GGA TTA GGA AAC AAT TTC AAT ATA TAT GTG GAA GCA TTT AAA GAA TGG GAA GAA GAT CCT AAT AAT CCA GCA ACC AGG ACC AGA GTA ATT

631
GAT CGC TTT CGT ATA CTT GAT GGG CTA CTT GAA AGG GAC ATT CCT TCG TTT CGA ATT TCT GGA TTT GAA GTA CCC CTT TTA TCC GTT TAT

721
GCT CAA GCG GCC AAT CTG CAT CTA GCT ATA TTA AGA GAT TCT GTA ATT TTT GGA GAA AGA TGG GGA TTG ACA ACG ATA AAT GTC AAT GAA

811
AAC TAT AAT AGA CTA ATT AGG CAT ATT GAT GAA TAT GCT GAT CAC TGT GCA AAT ACG TAT AAT CGG GGA TTA AAT AAT TTA CCG AAA TCT

901
ACG TAT CAA GAT TGG ATA ACA TAT AAT CGA TTA CGG AGA GAC TTA ACA TTG ACT GTA TTA GAT ATC GCC GCT TTC TTT CCA AAC TAT GAC

991
AAT AGG AGA TAT CCA ATT CAG CCA GTT GGT CAA CTA ACA AGG GAA GTT TAT ACG GAC CCA TTA ATT AAT TTT AAT CCA CAG TTA CAG TCT

1081
GTA GCT CAA TTA CCT ACT TTT AAC GTT ATG GAG AGC AGC GCA ATT AGA AAT CCT CAT TTA TTT GAT ATA TTG AAT AAT CTT ACA ATC TTT

1171
ACG GAT TGG TTT AGT GTT GGA CGC AAT TTT TAT TGG GGA GGA CAT CGA GTA ATA TCT AGC CTT ATA GGA GGT GGT AAC ATA ACA TCT CCT

1261
ATA TAT GGA AGA GAG GCG AAC CAG GAG CCT CCA AGA TCC TTT ACT TTT AAT GGA CCG GTA TTT AGG ACT TTA TCA ATT CCT ACT TTA CGA

1351
TTA TTA CAG CAA CCT TGC CAG CGC CAC CAT TTT AAT TTA CGT GGT GGT GAA GGA GTA GAA TTT TCT ACA CCT ACA AAT AGC TTT ACG TAT

1441
CGA GGA AGA GGT ACG GTT GAT TCT TTA ACT GAA TTA CCG CCT GAG GAT AAT AGT GTG CCA CCT CGC GAA GGA TAT AGT CAT CGT TTA TGT

1531
CAT GCA ACT TTT GTT CAA AGA TCT GGA ACA CCT TTT TTA ACA ACT GGT GTA GTA TTT TCT TGG ACG CAT CGT AGT GCA ACT CTT ACA AAT

1621
ACA ATT GAT CCA GAG AGA ATT AAT CAA ATA CCT TTA GTG AAA GGA TTT AGA GTT TGG GGG GGC ACC TCT GTC ATT ACA GGA CCA GGA TTT

1711
ACA GGA GGG GAT ATC CTT CGA AGA AAT ACC TTT GGT GAT TTT GTA TCT CTA CAA GTC AAT ATT AAT TCA CCA ATT ACC CAA AGA TAC CGT

1801
TTA AGA TTT CGT TAC GCT TCC AGT AGG GAT GCA CGA GTT ATA GTA TTA ACA GGA GCG GCA TCC ACA GGA GTG GGA GGC CAA GTT AGT GTA

9. Sequenz von Nukleotiden, die für ein Polypeptid kodiert, das in spezifischer Weise gegenüber Larven von Schmetterlingen der Familie der Nachtfalter, vorzugsweise gegenüber S. littoralis, toxisch ist, dadurch **gekennzeichnet,** daß

sie die in Anspruch 8 definierte Kette (I) oder (III) enthält.

EP 0 295 156 B1

**10.** Sequenz von Nukleotiden gemäß Anspruch 8 oder 9,
dadurch **gekennzeichnet,** daß
sie ein Initiationskodon ATG in Position 241 aufweist.

**11.** Sequenz gemäß jedem der Ansprüche 8 bis 10,
**gekennzeichnet** durch
eine Bindungsstelle an Ribosomen GGAGG in Positionen 230 bis 234.

**12.** Sequenz gemäß einem der Ansprüche 9 bis 11,
dadurch **gekennzeichnet,** daß
sie die Sequenz von Nukleotiden von Position 137 bis 177 (Position-103 bis -63 von oberhalb des Initiationskodons ATG) enthält, welche bezüglich ungefähr mindestens 70% der Region homolog ist, die oberhalb des Kristallgens des Stammes kurstaki-HD1 Dipel (BTK) vorliegt und die drei Promotoren BtI, BtII und Ec enthält, die in B. thuringiensis bzw. E. coli funktionell sind.

**13.** Sequenz von Nukleotiden,
dadurch **gekennzeichnet,** daß
sie für ein Polypeptid kodiert, das die Sequenz (II) der folgenden Aminosäuren enthält:

```
MET GLU GLU ASN ASN GLN ASN GLN CYS ILE PRO TYR ASN CYS LEU SER ASN

PRO GLU GLU VAL LEU LEU ASP GLY GLU ARG ILE SER THR GLY ASN SER SER ILE ASP ILE

SER LEU SER LEU VAL GLN PHE LEU VAL SER ASN PHE VAL PRO GLY GLY GLY PHE LEU VAL

GLY LEU ILE ASP PHE VAL TRP GLY ILE VAL GLY PRO SER GLN TRP ASP ALA PHE LEU VAL

GLN ILE GLU GLN LEU ILE ASN GLU ARG ILE ALA GLU PHE ALA ARG ASN ALA ALA ILE ALA

ASN LEU GLU GLY LEU GLY ASN ASN PHE ASN ILE TYR VAL GLU ALA PHE LYS GLU TRP GLU

GLU ASP PRO ASN ASN PRO GLU THR ARG THR ARG VAL ILE ASP ARG PHE ARG ILE LEU ASP

GLY LEU LEU GLU ARG ASP ILE PRO SER PHE ARG ILE SER GLY PHE GLU VAL PRO LEU LEU

SER VAL TYR ALA GLN ALA ALA ASN LEU HIS LEU ALA ILE LEU ARG ASP SER VAL ILE PHE

GLY GLU ARG TRP GLY LEU THR THR ILE ASN VAL ASN GLU ASN TYR ASN ARG LEU ILE ARG

HIS ILE ASP GLU TYR ALA ASP HIS CYS ALA ASN THR TYR ASN ARG GLY LEU ASN ASN LEU

PRO LYS SER THR TYR GLN ASP TRP ILE THR TYR ASN ARG LEU ARG ARG ASP LEU THR LEU

THR VAL LEU ASP ILE ALA ALA PHE PHE PRO ASN TYR ASP
```

oder dadurch **gekennzeichnet,** daß sie für ein Polypeptid kodiert, das die Sequenz (IV) der folgenden Aminosäuren enthält:

48

EP 0 295 156 B1

MET GLU GLU ASN ASN GLN ASN GLN CYS ILE

271 PRO TYR ASN CYS LEU SER ASN PRO GLU GLU VAL LEU LEU ASP GLY GLU ARG ILE SER THR GLY ASN SER SER ILE ASP ILE SER LEU SER

361 LEU VAL GLN PHE LEU VAL SER ASN PHE VAL PRO GLY GLY GLY PHE LEU VAL GLY LEU ILE ASP PHE VAL TRP GLY ILE VAL GLY PRO SER

451 GLN TRP ASP ALA PHE LEU VAL GLN ILE GLU GLN LEU ILE ASN GLU ARG ILE ALA GLU PHE ALA ARG ASN ALA ALA ILE ALA ASN LEU GLU

541 GLY LEU GLY ASN ASN PHE ASN ILE TYR VAL GLU ALA PHE LYS GLU TRP GLU GLU ASP PRO ASN ASN PRO ALA THR ARG THR ARG VAL ILE

631 ASP ARG PHE ARG ILE LEU ASP GLY LEU LEU GLU ARG ASP ILE PRO SER PHE ARG ILE SER GLY PHE GLU VAL PRO LEU LEU SER VAL TYR

721 ALA GLN ALA ALA ASN LEU HIS LEU ALA ILE LEU ARG ASP SER VAL ILE PHE GLY GLU ARG TRP GLY LEU THR THR ILE ASN VAL ASN GLU

811 ASN TYR ASN ARG LEU ILE ARG HIS ILE ASP GLU TYR ALA ASP HIS CYS ALA ASN THR TYR ASN ARG GLY LEU ASN ASN LEU PRO LYS SER

901 THR TYR GLN ASP TRP ILE THR TYR ASN ARG LEU ARG ARG ASP LEU THR LEU THR VAL LEU ASP ILE ALA ALA PHE PHE PRO ASN TYR ASP

991
ASN ARG ARG TYR PRO ILE GLN PRO VAL GLY GLN LEU THR ARG GLU VAL TYR THR ASP PRO LEU ILE ASN PHE ASN PRO GLN LEU GLN SER

1081
VAL ALA GLN LEU PRO THR PHE ASN VAL MET GLU SER SER ALA ILE ARG ASN PRO HIS LEU PHE ASP ILE LEU ASN ASN LEU THR ILE PHE

1171
THR ASP TRP PHE SER VAL GLY ARG ASN PHE TYR TRP GLY GLY HIS ARG VAL ILE SER SER LEU ILE GLY GLY GLY ASN ILE THR SER PRO

1261
ILE TYR GLY ARG GLU ALA ASN GLN GLU PRO PRO ARG SER PHE THR PHE ASN GLY PRO VAL PHE ARG THR LEU SER ILE PRO THR LEU ARG

1351
LEU LEU GLN GLN PRO CYS GLN ARG HIS HIS PHE ASN LEU ARG GLY GLY GLU GLY VAL GLU PHE SER THR PRO THR ASN SER PHE THR LYS

1441
ARG GLY ARG GLY THR VAL ASP SER LEU THR GLU LEU PRO PRO GLU ASP ASN SER VAL PRO PRO ARG GLU GLY TYR SER HIS ARG LEU CYS

1531
HIS ALA THR PHE VAL GLN ARG SER GLY THR PRO PHE LEU THR THR GLY VAL VAL PHE SER TRP THR HIS ARG SER ALA THR LEU THR ASN

1621
THR ILE ASP PRO GLU ARG ILE ASN GLN ILE PRO LEU VAL LYS GLY PHE ARG VAL TRP GLY GLY THR SER VAL ILE THR GLY PRO GLY PHE

1711
THR GLY GLY ASP ILE LEU ARG ARG ASN THR PHE GLY ASP PHE VAL SER LEU GLN VAL ASN ILE ASN SER PRO ILE THR GLN ARG TYR ARG

1801
LEU ARG PHE ARG TYR ALA SER SER ARG ASP ALA ARG VAL ILE VAL LEU THR GLY ALA ALA SER THR GLY VAL GLY GLY GLN VAL SER VAL

14. Rekombinanter Expressions- und Klonierungsvektor, enthaltend mindestens einen Teil der in jedem der Ansprüche 1 bis 13 definierten Nukleotidsequenz.

**15.** Plasmid gemäß Anspruch 14,
dadurch **gekennzeichnet,** daß
es sich um pHT671, wie dargestellt auf Figur 4, oder um pHT71 handelt, enthaltend ein DNA-Fragment HindIII-PstI aus einziglich DNA, die aus dem Stamm aizawai 7-29 stammt, der in einem Vektor pUC9 kloniert ist, welcher vorab mit den Enzymen HindIII und PstI hydrolysiert ist.

**16.** Modifizierte Bakterienstämme,
dadurch **gekennzeichnet,** daß
sie nach Transformation eine Sequenz von Nukleotiden gemäß einem der Ansprüche 1 bis 13 enthalten.

**17.** Bakterienstamm gemäß Anspruch 16,
dadurch **gekennzeichnet,** daß
er mindestens einen rekombinanten Vektor gemäß Anspruch 14 oder 15 enthält.

**18.** Polypeptid, das gegenüber Schmetterlingslarven und bevorzugt gegenüber S. littoralis toxisch ist,
dadurch **gekennzeichnet,** daß
es dazu befähigt ist, einen immunologischen Komplex mit Antikörpern zu bilden, die gegen Polypeptide der Sequenz (II) oder der Sequenz (IV) von Aminosäuren mit larvizider Wirksamkeit gegenüber S. littoralis gerichtet sind.

**19.** Polypeptid gemäß Anspruch 18,
dadurch **gekennzeichnet,** daß
es die Sequenz (II) oder die Sequenz (IV) der in Anspruch 13 definierten Aminosäuren enthält.

**20.** Verfahren zum Erhalt einer nukleotidischen Sequenz gemäß Anspruch 1, welche für mindestens einen Teil der N-terminalen Region eines Polypeptids kodiert, das in spezifischer Weise gegenüber Larven von Schmetterlingen der Familie der Nachtfalter, vorzugsweise gegenüber S. littoralis, toxisch ist, welches durch die folgenden Stufen gekennzeichnet ist:
- man führt eine Hybridisierung zwischen einerseits einer Sequenz von Nukleotiden eines gegen S. littoralis aktiven Stammes von B. thuringiensis und andererseits einem oder mehreren Sequenzen von Nukleotiden durch, welche als Sonden eingesetzt sind, welche aus dem Teil 5' einerseits und dem Teil 3' andererseits eines Restriktionsfragments eines Gens eines $\delta$-Endotoxins von B. thuringiensis, Stamm aizawai 7-29, stammen, wobei diese Teile 5' und 3' jeweils für den N-terminalen Teil und für den COOH-terminalen Teil eines Polypeptids von 130 kd kodieren, das gegenüber Schmetterlingen toxisch und insbesondere gegen P. brassicae wirksam und gegen S. littoralis unwirksam ist, wobei dieses Gen im in Fig. 2 dargestellten rekombinanten Plasmid pHTA2 kloniert worden ist,
- man isoliert das Fragment und
- kloniert es in einen Vektor, worauf seine Reinigung erfolgt.

**21.** Verfahren gemäß Anspruch 20,
dadurch **gekennzeichnet,** daß
das in der Klonierungsstufe an den Vektor rekombinierte Fragment aus mindestens einer Sequenz von Nukleotiden elaboriert ist, die aus mindestens einem rekombinanten Vektor hervorgegangen ist, der eine Nukleotidsequenz mindestens eines Stammes von B. thuringiensis enthält.

**22.** Verfahren gemäß Anspruch 21,
dadurch **gekennzeichnet,** daß
das in der Klonierungsstufe mit dem Vektor rekombinierte Fragment aus mehreren Nukleotidsequenzen elaboriert ist, die aus rekombinanten Vektoren hervorgegangen sind, die Nukleotidsequenzen von mindestens 2 von B. thuringiensis verschiedenen Stämmen enthalten, welche dieselben Restriktionskarten besitzen und ihrerseits eine ganze oder einen Teil der Nukleotidsequenzen enthalten, die dazu befähigt sind, für ein Polypeptid zu kodieren, das vorzugsweise gegenüber S. littoralis aktiv ist.

**23.** Verfahren gemäß Anspruch 21,
dadurch **gekennzeichnet,** daß
das in der Klonierungsstufe mit dem Vektor rekombinierte Fragment aus einem Restriktionsfragment HinIII-PstI elaboriert ist, welches aus dem Stamm aizawai 7-29 hervorgeht.

24. Verfahren gemäß Anspruch 22,
    dadurch **gekennzeichnet,** daß

    das in der Klonierungsstufe an den Vektor rekombinierte Fragment aus einem Restriktionsfragment HindIII-HincII elaboriert ist, das aus dem Stamm entomocidus 601 und aus einem Restriktionsfragment HincII - PstI stammt, welches aus dem Stamm aizawai 7-29 hervorgeht.

25. Verfahren gemäß Anspruch 20,
    dadurch **gekennzeichnet,** daß

    das gemäß Anspruch 23 rekombinierte Restriktionsfragment von einem in Fig. 1 dargestellten Plasmid pHTA6 und die gemäß Anspruch 24 rekombinierten Restriktionsfragmente HindIII-HincII und HincII-PstI von den rekombinanten Plasmiden pHTE6 bzw. pHTA6 gehalten sind, wobei die in Fig. 1 dargestellten genannten Plasmide pHTA6 und pHTE6 so vorliegen, wie sie mittels einer Sonde aus einem Fragment PvuII von 2 kb des Plasmids pBT15-88 isoliert sind, das dem inneren Teil eines chromosomischen Kristallgens des Stammes berliner 1715 entspricht, ausgehend von transformanten Klonen, die nukleotidische Sequenzen einschließen, welche aus Stämmen B. thuringiensis hervorgegangen sind, die gegenüber Larven von Schmetterlingen u.a. von S. littoralis wirksam sind.

26. Larvizide Zusammensetzung mit einer Wirksamkeit vorzugsweise gegenüber S. littoralis,
    dadurch **gekennzeichnet,** daß

    sie eine wirksame Menge von in jedem der Ansprüche 18 und 19 definierten Polypeptid enthält, das durch die nukleotidischen Sequenzen gemäß einem der Ansprüche 1 bis 13, durch den Vektor gemäß Anspruch 14 oder das Plasmid gemäß Anspruch 15 oder den bakteriellen Stamm gemäß jedem der Ansprüche 16 oder 17 exprimiert ist.

27. Anwendung der Sequenzen von Nukleotiden gemäß einem jeden der Ansprüche 1 bis 13 zur Erzeugung eines Polypeptids, das gegenüber Schmetterlingen, vorzugsweise S. littoralis, toxisch ist, in Mikroorganismen, die dazu befähigt sind, rekombinante Vektoren zu exprimieren, welche diejenigen Sequenzen wie E. coli, B. subtilis, B. cereus oder B. thuringiensis aufweisen.

28. Anwendung gemäß Anspruch 27,
    dadurch **gekennzeichnet,** daß

    die Sequenzen von Nukleotiden in Mikroorganismen eingebracht sind, die im Umfeld oder zusammen mit Pflanzen, wie Pseudomonas, Azospirillum oder Rhizobium, leben und dazu befähigt sind, diese Sequenzen aufweisende rekombinante Vektoren zu exprimieren.

29. Anwendung gemäß Anspruch 27 oder 28,
    dadurch **gekennzeichnet,** daß

    die Sequenzen von Nukleotiden in Mikroorganismen zusammen mit verschiedenen Genen von δ-Endotoxin eingebracht sind.

30. Anwendung von Sequenzen von Nukleotiden gemäß einem jeden der Ansprüche 1 bis 13 zur Transformation von gegenüber S. littoralis empfindlichen Pflanzen,
    dadurch **gekennzeichnet,** daß

    sie den Transfer und die Expression dieser Sequenzen in diesen Pflanzen umfaßt.

31. Pflanzliche Zellen, deren Genom, nach Transformation durch ein nicht-wesentlich biologisches Verfahren, in stabiler Form eine Sequenz von Nukleotiden besitzt, die dazu befähigt ist, ein gegenüber S. littoralis toxisches, in jedem der Ansprüche 1 bis 13 definiertes Polypeptid zu exprimieren, sowie aus deren Teilung hervorgegangene Zellen.

32. Pflanzen, die S. littoralis insbesondere zum Räuber haben, welche durch ein nicht-wesentlich biologisches Verfahren transformiert sind, deren Genom in stabiler Form eine in einem jeden der Ansprüche 1 bis 13 definierte Sequenz von Nukleotiden besitzt, welche dazu befähigt ist, ein gegenüber S. littoralis toxisches Polypeptid zu exprimieren, sowie Pflanzen, die aus deren Reproduktion, Vervielfältigung oder Kreuzungshybriden hervorgegangen sind.

**33.** Pflanze, die S. littoralis insbesondere zum Räuber hat, welche zusätzlich zu ihren genotypischen und phänotypischen Anfangsmerkmalen die Eigenschaft besitzt, ein bevorzugt gegenüber S. littoralis toxisches Polypeptid gemäß der in Anspruch 13 definierten Sequenz von Aminosäuren zu exprimieren, wobei diese Eigenschaft aus der in ihr Genom durch genetische Manipulation vorgenommenen Insertion einer zur Exprimierung des genannten Polypeptids befähigten Sequenz von Nukleotiden resultiert.

**34.** Korn, das dazu befähigt ist, eine Pflanze gemäß Anspruch 32 oder 33 zu ergeben, oder welches aus einer solchen Pflanze hervorgegangen ist,

dadurch **gekennzeichnet,** daß

es in seinem Genom durch genetische Manipulation eine Sequenz von Nukleotiden gemäß einem jeden der Ansprüche 1 bis 13 integriert enthält.

FIGURE 1

pHTA 6

pHTE 6

| B2 | : Bgl II | K | : Kpn I |
|----|----------|---|---------|
| E | : Eco R I | P1 | : Pst I |
| H2 | : Hinc II | P2 | : Pvu II |
| H | : Hind III | | |

1Kb

EP 0 295 156 B1

FIGURE 2

5'

Sonde I

Sonde 3

Sonde 2

pHTA2

HI    E         E    S    E  H    K    H         P         K

Gène de cristal

5' ⟶ 3'

1 Kb

E : EcoRI        K : KpnI

H : HindIII      P : PvuII

HI: HpaI         S : SalI

EP 0 295 156 B1

FIGURE 3

FIGURE 4

pHT 671

H2　　　　　　　　　　　　　H　　　　　H2　　　B2　　　K H　　P1

ADN du vecteur pUC9

ADN de la souche _entomocidus_ 601

ADN de la souche _aizawai_ 7-29

B2 : Bgl ll

H　: Hind lll

H2 : Hinc ll

K　: Kpn l

P1 : Pst l

1 Kb

EP 0 295 156 B1

FIGURE 5